(19) Europäisches Patentamt
European Patent Office
Office européen des brevets

(11) **EP 4 442 249 A1**

(12) **EUROPEAN PATENT APPLICATION**

(43) Date of publication:
**09.10.2024 Bulletin 2024/41**

(21) Application number: **23167083.7**

(22) Date of filing: **06.04.2023**

(51) International Patent Classification (IPC):
*A61K 9/00* (2006.01)    *A61K 31/5517* (2006.01)
*A61K 47/10* (2017.01)    *A61K 47/32* (2006.01)
*A61K 47/38* (2006.01)    *A61K 47/40* (2006.01)
*A61P 25/08* (2006.01)    *A61P 25/20* (2006.01)
*A61P 25/22* (2006.01)

(52) Cooperative Patent Classification (CPC):
**A61K 9/0056; A61K 9/006; A61K 31/5517;**
**A61K 47/32; A61K 47/38; A61K 47/40;**
**A61P 25/08;** A61K 47/10

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **B. Braun Melsungen AG**
**34212 Melsungen (DE)**

(72) Inventors:
• **Janas, Christine**
**34212 Melsungen (DE)**

• **Porredon Guarch, Constanca**
**34212 Melsungen (DE)**
• **Ziller, Antje**
**56626 Andernach (DE)**
• **Bauer, Marius**
**56626 Andernach (DE)**

(74) Representative: **Maiwald GmbH**
**Elisenhof**
**Elisenstraße 3**
**80335 München (DE)**

(54) **UNIT DOSAGE COMPOSITION COMPRISING MIDAZOLAM**

(57)    The present invention relates to a unit dosage composition comprising midazolam or a pharmaceutically acceptable salt thereof, a cyclodextrin, and at least one matrix material, as well as to the use of said unit dosage composition in medicine and therapy, in particular in the treatment of febrile seizure, epileptic seizure, such as status epilepticus, fear, such as preoperative anxiolysis to reduce fear, and/or agitation, such as agitation prior to surgery.

Fig. 2

## Description

### Field of the invention

The present invention relates to a unit dosage composition comprising midazolam or a pharmaceutically acceptable salt thereof, a cyclodextrin, and at least one matrix material, as well as to the use of said unit dosage composition in medicine and therapy.

### Background

Midazolam is a short-acting, hypnotic, and sedative drug with muscle relaxant properties within the drug class of benzodiazepines.

Midazolam acts on nerve cells in the brain and is used to control convulsions (fits or seizures) in children and adults with epilepsy or other diseases associated with seizures, or in children with febrile seizures.

The brain and nerves are made up of many nerve cells that communicate with each other through electrical signals. These signals must be carefully regulated for the brain and nerves to function properly. When abnormally rapid and repetitive electrical signals are evoked in the brain, it becomes over-stimulated and normal function is disturbed. This results in fits or seizures. Midazolam works by amplifying the action of the neurotransmitter GABA in the brain. GABA is involved in transmitting messages between the nerve cells and acts as a natural "nerve-calming" agent. It helps to reduce the nerve activity in the brain, to maintain it in balance, and is involved in inducing sleepiness, reducing anxiety and relaxing muscles.

Midazolam is commercially available in the form of its hydrochloride salt, for example in the form of a glycerine-based syrup sold under the trade name VERSED®, which contains 2.5 mg/mL of midazolam. Midazolam is also marketed in the form of its maleate salt, for example in tablets containing 7.5 or 15 mg per tablet under the trademark DORMICUM®. Products formulated for administration via the buccal route are EPISTATUS® (10 mg/mL midazolam in liquid form) and BUCCOLAM® (2.5mg/0.5 mL, 5 mg/mL, 7.5 mg/mL or 10 mg/mL midazolam in liquid form).

Compositions of midazolam for oral administration (such as tablets, and syrups) are primarily used for epileptic and other seizures, additionally as pre anaesthetic medication to reduce fear and agitation prior to surgeries. On the other hand, intravenous (IV) formulations of midazolam are used for sedative purposes both in the operating room (OR), the intensive care unit (ICU) and for procedural sedation.

It is understood that the bioavailability of per-oral dosage forms of midazolam differs from plasma profiles achieved after intravenous administration. While the intravenous administration offers immediate therapeutic plasma levels followed by a decline in plasma concentration according to the elimination kinetics, the per-oral administration achieves maximal plasma levels ($C_{max}$) typically at 1 to 2 hours after swallowing the tablet. Furthermore, orally administered Midazolam is subjected to first-pass metabolism resulting in a reduction in the plasma concentration of the drug and reduced bioavailability.

Hence, both routes are not suitable for administering midazolam in a pre-operative setting: To the contrary, patients receiving midazolam per-orally prior to surgery frequently show some kind of hang-over effect, and the decline in plasma levels is prolonged due to slow resorption of the drug from the small intestine. This results in prolonged sedation of patients due to midazolam plasma levels and increased recovery times after surgery making a rapid discharge from the wake-up or recovery room rather impossible.

Whilst IV administration does not show similar limitations, an IV access needs to be available and timing between midazolam administration and start of the surgery or intervention needs to be controlled in order not to lose the anxiolytic effect before the surgery starts.

Thus, both dosage forms, the intravenous solutions and per-oral tablets, do not offer the appropriate pharmacokinetic profile of the drug specifically for patients in short interventions or surgeries.

Buccally administered midazolam shows a rapid absorption. Furthermore, the achieved plasma levels are sufficient to provide therapeutic effect in the patient. However, in view of the importance of rapid delivery of midazolam to patients in need thereof, it would be advantageous if the delivery of the drug could be safer, more simplified and if a lower dose could be used. A lower dose leading to the desired therapeutic effect could reduce frequency and seriousness of side-effects such as, e.g., respiratory depression.

However, no suitable formulation with appropriate dosing and tolerable taste masking is available until now.

### Summary

The inventors of the present disclosure have surprisingly found that midazolam, or a pharmaceutically acceptable salt thereof, can be formulated into a unit dosage composition with a cyclodextrin and at least one matrix material to provide for a rapid absorption of midazolam or a pharmaceutically acceptable salt thereof through the oral mucosa and controlled metabolism and excretion. Additionally, the typical, bitter taste of midazolam or a pharmaceutically acceptable salt thereof, that limits the tolerability of midazolam for oral administration, can be significantly reduced. The solubility in water is also increased by the use of cyclodextrins in connection with midazolam, and it is assumed that absorption of midazolam is thereby increased and/or accelerated.

According to a first aspect, the present disclosure relates to a unit dosage composition in form of an oral thin film comprising midazolam or a pharmaceutically acceptable salt thereof, a cyclodextrin, and at least one matrix material.

According to a second aspect, the present disclosure relates to a method for manufacturing the unit dosage composition according to the first aspect of the present disclosure, comprising the steps of

a) providing an aqueous solution comprising midazolam or a pharmaceutically acceptable salt thereof, a cyclodextrin, and at least one matrix material,
b) coating the solution on a liner, and
c) allowing the solution to dry and form a film.

According to a third aspect, the present disclosure relates to a unit dosage composition according to the first aspect of the present disclosure for use in the treatment of febrile seizure, epileptic seizure, such as status epilepticus, fear, such as preoperative anxiolysis to reduce fear, and/or agitation, such as agitation prior to surgery.

According to a fourth aspect, the present disclosure relates to a method of treatment of febrile seizure, epileptic seizure, such as status epilepticus, fear, such as preoperative anxiolysis to reduce fear, and/or agitation, such as agitation prior to surgery, wherein the unit dosage composition according to the first aspect of the present disclosure is administered to a subject.

According to a fifth aspect, the present disclosure relates to a use of the unit dosage composition according to the first aspect of the present disclosure in the manufacture of a medicament for the treatment of febrile seizure, epileptic seizure, such as status epilepticus, fear, such as preoperative anxiolysis to reduce fear, and/or agitation, such as agitation prior to surgery.

Brief description of the figures

Fig. 1:     shows the $T_{max}$ of midazolam after administration of the oral thin films of example 1, Dormicum® tablet with 7.5 mg midazolam, and solution of midazolam.

Fig. 2:     shows the $C_{max}$ of midazolam after administration of the oral thin films of example 1, Dormicum® tablet with 7.5 mg midazolam, and solution of midazolam.

Fig. 3:     shows the mean midazolam plasma concentration vs. time after administration of the oral thin film "PVA foam" according to example 1 in comparison to Dormicum® tablet and Midazolam B. Braun 5 mg/mL solution for injection/infusion.

Fig. 4:     shows the mean midazolam plasma concentration vs. time after administration of the oral thin film "PVA film" according to example 1 in comparison to a Dormicum® tablet and Midazolam B. Braun 5 mg/mL solution for injection/infusion.

Fig. 5:     shows the mean midazolam plasma concentration vs. time after administration of the oral thin film "HPMC film pH 4.0" according to example 1 in comparison to a Dormicum® tablet and Midazolam B. Braun 5 mg/mL solution for injection/infusion.

Fig. 6:     shows the mean midazolam plasma concentration vs. time after administration of the oral thin film "HPMC film pH 5.0" according to example 1 in comparison to a Dormicum® tablet and Midazolam B. Braun 5 mg/mL solution for injection/infusion.

**Detailed Description**

The invention of the present disclosure is described in the following in more detail, exemplified by preferred embodiments and embodiment examples. However, it is understood that the scope of the present disclosure is not limited thereto, but only by the appendant claims.

The present disclosure, in very general terms, relates to five aspects, namely a first aspect being directed to a unit dosage composition, a second aspect being directed to a method for manufacturing said unit dosage composition, a third aspect being directed to said unit dosage composition for use in the treatment or prevention of febrile seizure, acute seizure, epileptic seizure, such as status epilepticus, fear, such as preoperative anxiolysis to reduce fear, and/or agitation, such as agitation prior to surgery, a fourth aspect being directed to a method of treatment of such disease by administering said unit dosage composition, and a fifth aspect being directed to a use of said unit dosage composition for the manufacture of a medicament.

The unit dosage composition

According to a first aspect, the present disclosure relates to a unit dosage composition in form of an oral thin film comprising midazolam or a pharmaceutically acceptable salt thereof, a cyclodextrin and at least one matrix material.

The inventors of the present disclosure have surprisingly found that unit dosage compositions comprising midazolam or a pharmaceutically acceptable salt thereof, a cyclodextrin, and at least one matrix material provide for a rapid absorption of the midazolam or pharmaceutically acceptable salt thereof through the oral mucosa and controlled metabolism and excretion. Additionally, the typical, bitter taste of midazolam or a pharmaceutically acceptable salt thereof - that limits the suitability of midazolam for use in the absorption via the oral mucosa - can be significantly reduced to open up new ways of fast and safe treatments with midazolam.

According to the present disclosure, the unit dosage composition is in the form of an oral thin film. According to a preferred embodiment of the present disclosure, the unit dosage composition is in the form of a buccal film. Such oral thin film is typically a paper-thin polymer film that is used as a carrier for drug substance(s).

According to a preferred embodiment of the present disclosure, said oral thin film has a thickness of 2,000 $\mu$m or less. According to another preferred embodiment of the present disclosure, said oral thin film has a thickness of 1,500 $\mu$m or less. According to another preferred embodiment of the present disclosure, said oral thin film has a thickness of 1,000 $\mu$m or less. According to another preferred embodiment of the present disclosure, said oral thin film has a thickness of 800 $\mu$m or less. According to another preferred embodiment of the present disclosure, said oral thin film has a thickness of 700 $\mu$m or less. According to another preferred embodiment of the present disclosure, said oral thin film has a thickness of 600 $\mu$m or less. According to a further preferred embodiment of the present disclosure, said oral thin film has a thickness of 500 $\mu$m or less. It is preferred to have thinner films as they are more flexible and easier to apply, as well as providing a smoother feeling or texture in the mouth. Furthermore, thinner films are typically dissolved faster and therefor enable faster absorption. On the other hand, if the film is too thin, it may become more difficult to apply, or it may even be destroyed due to lower stability.

According to another preferred embodiment of the present disclosure, the oral thin film has a thickness in the range of from about 10 $\mu$m to about 1,000 $\mu$m. According to another preferred embodiment of the present disclosure, the oral thin film has a thickness in the range of from about 30 $\mu$m to about 800 $\mu$m. According to another preferred embodiment of the present disclosure, the oral thin film has a thickness in the range of from about 40 $\mu$m to about 700 $\mu$m. According to another preferred embodiment of the present disclosure, the oral thin film has a thickness in the range of from about 50 $\mu$m to about 600 $\mu$m. According to a further preferred embodiment of the present disclosure, the oral thin film has a thickness in the range of from about 50 $\mu$m to about 500 $\mu$m.

It is understood that an oral thin film is administered into the oral cavity, such as on the tongue, under the tongue (sublingually) or applied to the cheek. Upon contact with saliva, an oral thin film dissolves rapidly and the drug is released into the oral cavity. In other words, the administration of an oral thin film does not require water or swallowing.

In the oral cavity, the drug is absorbed into the blood stream through the oral mucosa that is a mucous membrane lining the inside of the mouth. The oral mucosal epithelium is a 40-50 cell layer called mucus that is made up of carbohydrates and proteins. The mucosal thickness at the mouth base, tongue, and gums ranges from 100 to 200 $\mu$m.

According to a preferred embodiment of the present disclosure, the unit dosage composition is for oral administration. According to a further preferred embodiment of the present disclosure, the unit dosage composition is for buccal administration. According to a further preferred embodiment of the present disclosure, the unit dosage composition is for sublingual administration.

Buccal films and oral thin films (OTFs) are solid dosage forms following the Pharmacopoeia European monograph "Oromucosal preparations", more specifically the section on "Orodispersible films" within this monograph.

Midazolam is a well-known drug substance with sedative, anxiolytic, amnesic and hypnotic properties. Hence, midazolam, or pharmaceutically acceptable salts thereof are used in the treatment of various diseases, including anxiety and fear, procedural sedation, preoperative sedation, for the induction of general anesthesia, for sedation of people who are ventilated in critical care units, the acute management of agitation (e.g., in the acute management of schizophrenia when it is associated with aggressive or out-of-control behavior; or in preoperative situations), in the final stages of end-of-life care, and seizures, such as status epilepticus.

In any of such situations, oral administration of midazolam, particularly in the form of buccal films and oral thin films is favored - not only for non-professionals. Such dosage forms provide the advantage that there is no need for the patient to swallow the unit dosage composition in an emergency situation - which is particularly difficult for patients who do not have control over their muscle functions or in severe psychotic conditions. Instead, it is sufficient to place the buccal film or oral thin film in the mouth of a patient, where the midazolam is absorbed through the oral mucosa. Thus, not only the first-pass effect is avoided, but also a fast onset of action is achieved by the unit dosage compositions according to the present disclosure.

Furthermore, the unit dosage composition according to the present disclosure is solid. Such solid unit dosage compositions have the advantage over liquid dosage forms that the solid unit dosage composition according to the present disclosure cannot be swallowed accidentally. Furthermore, such solid unit dosage composition does not - other than liquid compositions, such as syrups - flow out of the mouth, once the solid unit dosage composition is placed in the mouth of the patient in need of treatment.

*Midazolam*

The amount of midazolam may be determined according to the needs of the patient, and in accordance with usual dosing amounts for midazolam. The following amounts of midazolam in the unit dosage composition of the present disclosure relate to the amount of midazolam determined on the basis of midazolam free base.

According to a preferred embodiment of the present disclosure, the unit dosage composition comprises an amount of midazolam in the range of from about 0.01 mg to about 50 mg. According to another preferred embodiment of the present disclosure, the unit dosage composition comprises an amount of midazolam in the range of from about 0.1 mg to about 20 mg. According to another preferred embodiment of the present disclosure, the unit dosage composition comprises an amount of midazolam in the range of from about 0.5 mg to about 10 mg. According to another preferred embodiment of the present disclosure, the unit dosage composition comprises an amount of midazolam in the range of from about 1 mg to about 5 mg. According to another preferred embodiment of the present disclosure, the unit dosage composition comprises an amount of midazolam in the range of from about 1.5 mg to about 4.5 mg. According to another preferred embodiment of the present disclosure, the unit dosage composition comprises an amount of midazolam in the range of from about 1.8 mg to about 4.3 mg. According to another preferred embodiment of the present disclosure, the unit dosage composition comprises an amount of midazolam in the range of from about 2.0 mg to about 4.0 mg. According to another preferred embodiment of the present disclosure, the unit dosage composition comprises an amount of midazolam in the range of from about 2.3 mg to about 3.5 mg. According to another preferred embodiment of the present disclosure, the unit dosage composition comprises an amount of midazolam in the range of from about 2.3 mg to about 3.2 mg.

According to a further preferred embodiment of the present disclosure, the unit dosage composition comprises an amount of midazolam in the range of from about 2 mg to about 4 mg. According to a further preferred embodiment of the present disclosure, the unit dosage composition comprises an amount of midazolam in the range of from about 2.4 mg to about 3.1 mg. According to a further preferred embodiment of the present disclosure, the unit dosage composition comprises an amount of midazolam in the range of from about 2.5 mg to about 3.0 mg. According to a further preferred embodiment of the present disclosure, the unit dosage composition comprises an amount of midazolam in the range of from about 2.6 mg to about 2.9 mg.

According to another preferred embodiment of the present disclosure, the unit dosage composition comprises an amount of midazolam of about 0.01 mg or more. According to another preferred embodiment of the present disclosure, the unit dosage composition comprises an amount of midazolam of about 0.1 mg or more. According to another preferred embodiment of the present disclosure, the unit dosage composition comprises an amount of midazolam of about 0.2 mg or more. According to another preferred embodiment of the present disclosure, the unit dosage composition comprises an amount of midazolam of about 0.5 mg or more. According to another preferred embodiment of the present disclosure, the unit dosage composition comprises an amount of midazolam of about 1 mg or more. According to another preferred embodiment of the present disclosure, the unit dosage composition comprises an amount of midazolam of about 1.5 mg or more. According to another preferred embodiment of the present disclosure, the unit dosage composition comprises an amount of midazolam of about 1.8 mg or more. According to another preferred embodiment of the present disclosure, the unit dosage composition comprises an amount of midazolam of about 2.0 mg or more. According to another preferred embodiment of the present disclosure, the unit dosage composition comprises an amount of midazolam of about 2.3 mg or more. According to another preferred embodiment of the present disclosure, the unit dosage composition comprises an amount of midazolam of about 2.4 mg or more. According to another preferred embodiment of the present disclosure, the unit dosage composition comprises an amount of midazolam of about 2.5 mg or more. According to a further preferred embodiment of the present disclosure, the unit dosage composition comprises an amount of midazolam of about 2.6 mg or more.

According to a further preferred embodiment of the present disclosure, the unit dosage composition comprises an amount of midazolam of about 2.0 mg or more. According to a further preferred embodiment of the present disclosure, the unit dosage composition comprises an amount of midazolam of about 2.5 mg or more.

According to another preferred embodiment of the present disclosure, the unit dosage composition comprises an amount of midazolam of about 50 mg or less. According to another preferred embodiment of the present disclosure, the unit dosage composition comprises an amount of midazolam of about 20 mg or less. According to another preferred embodiment of the present disclosure, the unit dosage composition comprises an amount of midazolam of about 15 mg or less. According to another preferred embodiment of the present disclosure, the unit dosage composition comprises an amount of midazolam of about 10 mg or less. According to another preferred embodiment of the present disclosure, the unit dosage composition comprises an amount of midazolam of about 5 mg or less. According to another preferred embodiment of the present disclosure, the unit dosage composition comprises an amount of midazolam of about 4.5 mg or less. According to another preferred embodiment of the present disclosure, the unit dosage composition comprises an amount of midazolam of about 4.3 mg or less. According to another preferred embodiment of the present disclosure, the unit dosage composition comprises an amount of midazolam of about 4.0 mg or less. According to another preferred embodiment of the present disclosure, the unit dosage composition comprises an amount of midazolam of about 3.5 mg or less. According to another preferred embodiment of the present disclosure, the unit dosage composition comprises an amount of midazolam of about 3.2 mg or less. According to another preferred embodiment of the present disclosure, the unit dosage composition comprises an amount of midazolam of about 3.1 mg or less. According to another preferred embodiment of the present disclosure, the unit dosage composition comprises an amount of midazolam of about 3.0 mg or less. According to another preferred embodiment of the present disclosure, the unit dosage composition comprises an amount of midazolam of about 2.9 mg or less.

According to a further preferred embodiment of the present disclosure, the unit dosage composition comprises an amount of midazolam of about 4 mg or less. According to a further preferred embodiment of the present disclosure, the unit dosage composition comprises an amount of midazolam of about 3.5 mg or less. According to a further preferred embodiment of the present disclosure, the unit dosage composition comprises an amount of midazolam of about 3.0 mg or less. According to a further preferred embodiment of the present disclosure, the unit dosage composition comprises an amount of midazolam of about 2.9 mg or less.

According to a preferred embodiment of the present disclosure, the unit dosage composition comprises midazolam, or a pharmaceutically acceptable salt thereof in a molar amount in the range of from about 0.03 $\mu$mol to about 153.48 $\mu$mol. According to another preferred embodiment of the present disclosure, the unit dosage composition comprises midazolam, or a pharmaceutically acceptable salt thereof in a molar amount in the range of from about 0.31 $\mu$mol to about 61.39 $\mu$mol. According to another preferred embodiment of the present disclosure, the unit dosage composition comprises midazolam, or a pharmaceutically acceptable salt thereof in a molar amount in the range of from about 0.61 $\mu$mol to about 46.04 $\mu$mol. According to another preferred embodiment of the present disclosure, the unit dosage composition comprises midazolam, or a pharmaceutically acceptable salt thereof in a molar amount in the range of from about 1.53 $\mu$mol to about 30.70 $\mu$mol. According to another preferred embodiment of the present disclosure, the unit dosage composition comprises midazolam, or a pharmaceutically acceptable salt thereof in a molar amount in the range of from about 3.07 $\mu$mol to about 15.35 $\mu$mol. According to another preferred embodiment of the present disclosure, the unit dosage composition comprises midazolam, or a pharmaceutically acceptable salt thereof in a molar amount in the range of from about 4.60 $\mu$mol to about 13.81 $\mu$mol. According to another preferred embodiment of the present disclosure, the unit dosage composition comprises midazolam, or a pharmaceutically acceptable salt thereof in a molar amount in the range of from about 5.53 $\mu$mol to about 13.20 $\mu$mol. According to another preferred embodiment of the present disclosure, the unit dosage composition comprises midazolam, or a pharmaceutically acceptable salt thereof in a molar amount in the range of from about 6.14 $\mu$mol to about 12.28 $\mu$mol. According to another preferred embodiment of the present disclosure, the unit dosage composition comprises midazolam, or a pharmaceutically acceptable salt thereof in a molar amount in the range of from about 7.06 $\mu$mol to about 10.74 $\mu$mol. According to another preferred embodiment of the present disclosure, the unit dosage composition comprises midazolam, or a pharmaceutically acceptable salt thereof in a molar amount in the range of from about 7.06 $\mu$mol to about 9.82 $\mu$mol. According to another preferred embodiment of the present disclosure, the unit dosage composition comprises midazolam, or a pharmaceutically acceptable salt thereof in a molar amount in the range of from about 7.37 $\mu$mol to about 9.52 $\mu$mol. According to another preferred embodiment of the present disclosure, the unit dosage composition comprises midazolam, or a pharmaceutically acceptable salt thereof in a molar amount in the range of from about 7.67 $\mu$mol to about 9.21 $\mu$mol. According to another preferred embodiment of the present disclosure, the unit dosage composition comprises midazolam, or a pharmaceutically acceptable salt thereof in a molar amount in the range of from about 7.98 $\mu$mol to about 8.90 $\mu$mol.

According to a further another preferred embodiment of the present disclosure, the unit dosage composition comprises midazolam, or a pharmaceutically acceptable salt thereof in a molar amount in the range of from about 6.14 $\mu$mol to about 12.28 $\mu$mol. According to a further another preferred embodiment of the present disclosure, the unit dosage composition comprises midazolam, or a pharmaceutically acceptable salt thereof in a molar amount in the range of from about 7.06 $\mu$mol to about 9.82 $\mu$mol. According to a further another preferred embodiment of the present disclosure, the unit dosage composition comprises midazolam, or a pharmaceutically acceptable salt thereof in a molar amount in the range of from about 7.67 $\mu$mol to about 9.21 $\mu$mol. According to a further another preferred embodiment of the present disclosure, the unit dosage composition comprises midazolam, or a pharmaceutically acceptable salt thereof in a molar amount in the range of from about 6.98 $\mu$mol to about 8.90 $\mu$mol.

According to a preferred embodiment of the present disclosure, the unit dosage composition comprises midazolam, or a pharmaceutically acceptable salt thereof in a molar amount of about 0.03 $\mu$mol or more. According to another preferred embodiment of the present disclosure, the unit dosage composition comprises midazolam, or a pharmaceutically acceptable salt thereof in a molar amount of about 0.31 $\mu$mol or more. According to another preferred embodiment of the present disclosure, the unit dosage composition comprises midazolam, or a pharmaceutically acceptable salt thereof in a molar amount of about 0.61 $\mu$mol or more. According to another preferred embodiment of the present disclosure, the unit dosage composition comprises midazolam, or a pharmaceutically acceptable salt thereof in a molar amount of about 1.53 $\mu$mol or more. According to another preferred embodiment of the present disclosure, the unit dosage composition comprises midazolam, or a pharmaceutically acceptable salt thereof in a molar amount of about 3.07 $\mu$mol or more. According to another preferred embodiment of the present disclosure, the unit dosage composition comprises midazolam, or a pharmaceutically acceptable salt thereof in a molar amount of about 4.60 $\mu$mol or more. According to another preferred embodiment of the present disclosure, the unit dosage composition comprises midazolam, or a pharmaceutically acceptable salt thereof in a molar amount of about 5.53 $\mu$mol or more. According to another preferred embodiment of the present disclosure, the unit dosage composition comprises midazolam, or a pharmaceutically acceptable salt thereof in a molar amount of about 6.14 $\mu$mol or more. According to another preferred embodiment of the present disclosure, the unit dosage composition comprises midazolam, or a pharmaceutically acceptable salt thereof in a molar amount of about 7.06 $\mu$mol or more. According to another preferred embodiment of the present disclosure, the unit dosage composition comprises midazolam, or a pharmaceutically acceptable salt thereof in a molar amount of about 7.37 $\mu$mol or more. According to another preferred embodiment of the present disclosure, the unit dosage composition comprises midazolam, or a pharmaceutically acceptable salt thereof in a molar amount of about 7.67 $\mu$mol or more. According to another preferred embodiment of the present disclosure, the unit dosage composition comprises midazolam, or a pharmaceutically acceptable salt thereof in a molar amount of about 7.98 $\mu$mol or more.

According to a further another preferred embodiment of the present disclosure, the unit dosage composition comprises midazolam, or a pharmaceutically acceptable salt thereof in a molar amount of about 6.14 $\mu$mol or more. According to a further another preferred embodiment of the present disclosure, the unit dosage composition comprises midazolam, or a pharmaceutically acceptable salt thereof in a molar amount of about 7.06 $\mu$mol or more. According to a further another preferred embodiment of the present disclosure, the unit dosage composition comprises midazolam, or a pharmaceutically acceptable salt thereof in a molar amount of about 7.67 $\mu$mol or more. According to a further another preferred embodiment of the present disclosure, the unit dosage composition comprises midazolam, or a pharmaceutically acceptable salt thereof in a molar amount of about 6.98 $\mu$mol or more.

According to a preferred embodiment of the present disclosure, the unit dosage composition comprises midazolam, or a pharmaceutically acceptable salt thereof in a molar amount of about 153.48 $\mu$mol or less. According to another preferred embodiment of the present disclosure, the unit dosage composition comprises midazolam, or a pharmaceutically acceptable salt thereof in a molar amount of about 61.39 $\mu$mol or less. According to another preferred embodiment of the present disclosure, the unit dosage composition comprises midazolam, or a pharmaceutically acceptable salt thereof in a molar amount of about 46.04 $\mu$mol or less. According to another preferred embodiment of the present disclosure, the unit dosage composition comprises midazolam, or a pharmaceutically acceptable salt thereof in a molar amount of about 30.70 $\mu$mol or less. According to another preferred embodiment of the present disclosure, the unit dosage composition comprises midazolam, or a pharmaceutically acceptable salt thereof in a molar amount of about 15.35 $\mu$mol or less. According to another preferred embodiment of the present disclosure, the unit dosage composition comprises midazolam, or a pharmaceutically acceptable salt thereof in a molar amount of about 13.81 $\mu$mol or less. According to another preferred embodiment of the present disclosure, the unit dosage composition comprises midazolam, or a pharmaceutically acceptable salt thereof in a molar amount of about 13.20 $\mu$mol. According to another preferred embodiment of the present disclosure, the unit dosage composition comprises midazolam, or a pharmaceutically acceptable salt thereof in a molar amount of about 12.28 $\mu$mol or less. According to another preferred embodiment of the present disclosure, the unit dosage composition comprises midazolam, or a pharmaceutically acceptable salt thereof in a molar amount of about 10.74 $\mu$mol or less. According to another preferred embodiment of the present disclosure, the unit dosage composition comprises midazolam, or a pharmaceutically acceptable salt thereof in a molar amount of about 9.82 $\mu$mol or less. According to another preferred embodiment of the present disclosure, the unit dosage composition comprises midazolam, or a pharmaceutically acceptable salt thereof in a molar amount of about 9.52 $\mu$mol or less. According to another preferred embodiment of the present disclosure, the unit dosage composition comprises midazolam, or a pharmaceutically acceptable salt thereof in a molar amount of about 9.21 $\mu$mol or less. According to another preferred embodiment of the present disclosure, the unit dosage composition comprises midazolam, or a pharmaceutically acceptable salt thereof in a molar amount of about 8.90 $\mu$mol or less.

According to a further another preferred embodiment of the present disclosure, the unit dosage composition comprises midazolam, or a pharmaceutically acceptable salt thereof in a molar amount of about 12.28 $\mu$mol or less.

According to a further another preferred embodiment of the present disclosure, the unit dosage composition comprises midazolam, or a pharmaceutically acceptable salt thereof in a molar amount of about $9.82\,\mu$mol or less. According to a further another preferred embodiment of the present disclosure, the unit dosage composition comprises midazolam, or a pharmaceutically acceptable salt thereof in a molar amount of about $9.21\,\mu$mol or less. According to a further another preferred embodiment of the present disclosure, the unit dosage composition comprises midazolam, or a pharmaceutically acceptable salt thereof in a molar amount of about $8.90\,\mu$mol or less.

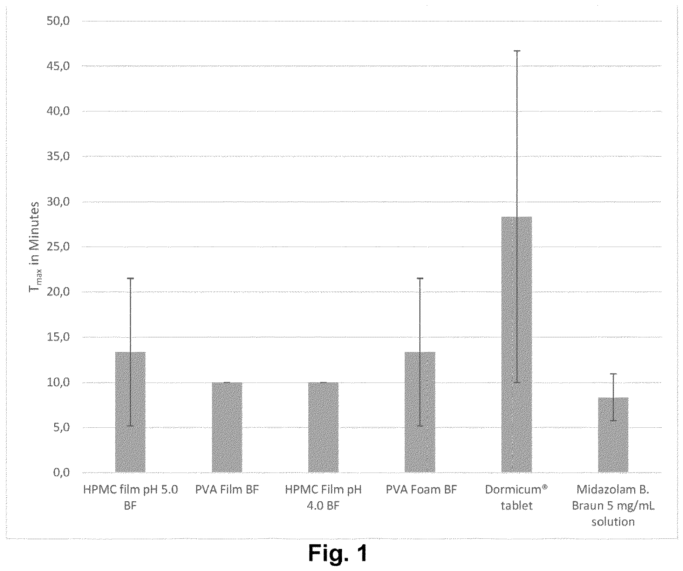

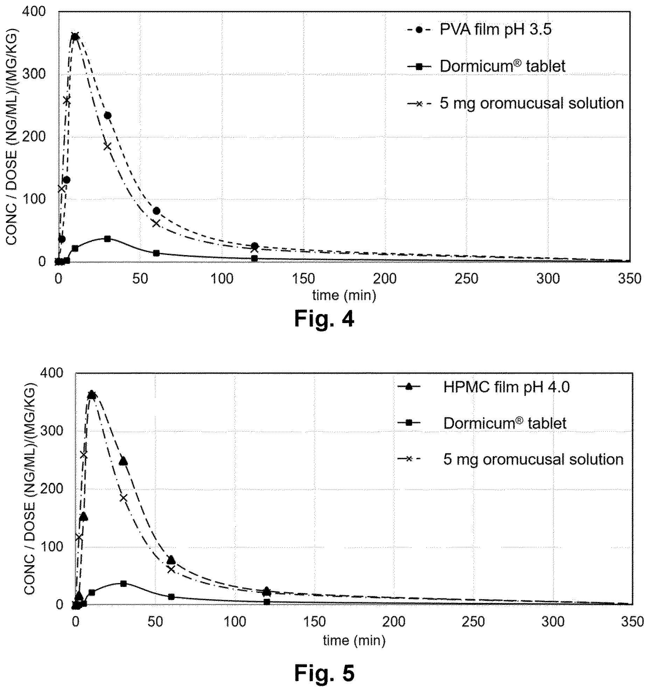

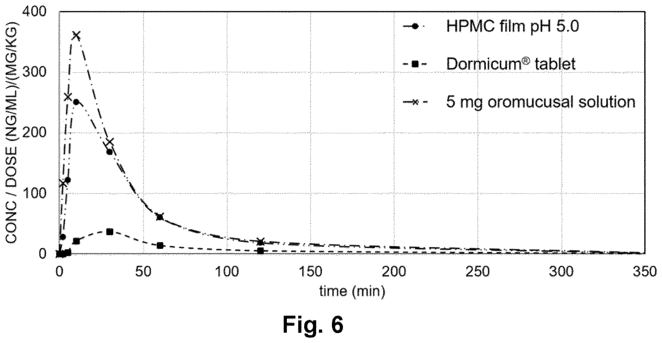

The inventors of the present disclosure have surprisingly found that the unit dosage compositions according to the present disclosure can efficiently deliver even small amounts of midazolam, such as 2.7 mg of midazolam, or a pharmaceutically acceptable salt thereof, to the blood stream achieving pharmaceutically efficient plasma concentrations shortly after administration. As shown in example 3 and Figures 1 to 6 of the present disclosure, the unit dosage compositions according to the present disclosure provide for plasma concentrations of from about 80 ng/mL to about 120 ng/mL when administered to Marshall dogs. Although the investigated formulations contain only 3 mg of midazolam hydrochloride, the maximum plasma concentration $C_{max}$ is considerably higher than the maximum plasma concentration $C_{max}$ obtained after the administration of Dormicum® tablets although said tablets contain a much higher dose of midazolam. Additionally, the maximum plasma concentration $C_{max}$ of the unit dosage compositions of the present disclosure is in a similar range compared to midazolam administered from a 5 mg/mL solution.

According to a preferred embodiment of the present disclosure, the unit dosage composition comprises midazolam. According to another preferred embodiment of the present disclosure, the unit dosage composition comprises midazolam maleate. According to a further preferred embodiment of the present disclosure, the unit dosage composition comprises midazolam hydrochloride.

## The cylcodextrin

According to the first aspect of the present disclosure, the unit dosage composition comprises a cyclodextrin. The cyclodextrin can be unmodified or a derivative thereof.

According to a preferred embodiment of the present disclosure, the cyclodextrin is selected from the group consisting of alpha-cyclodextrin, beta-cyclodextrin, gamma-cyclodextrin, and delta-cyclodextrin. According to another preferred embodiment of the present disclosure, the cyclodextrin is selected from the group consisting a beta-cyclodextrin, gamma-cyclodextrin, and delta-cyclodextrin.

According to another preferred embodiment of the present disclosure, the cyclodextrin is a beta-cyclodextrin. According to another preferred embodiment of the present disclosure, the cyclodextrin is a gamma-cyclodextrin. According to another preferred embodiment of the present disclosure, the cyclodextrin is a delta-cyclodextrin.

According to a further preferred embodiment of the present disclosure, the cyclodextrin is permethyl-beta-cyclodextrin or hydroxypropyl-beta-cyclodextrin (HPbCD). According to a further preferred embodiment of the present disclosure, the cyclodextrin is hydroxypropyl-beta-cyclodextrin (HPbCD).

It is understood that cyclodextrins have the possibility to bind other molecules in their quasi-cylindrical interiors.

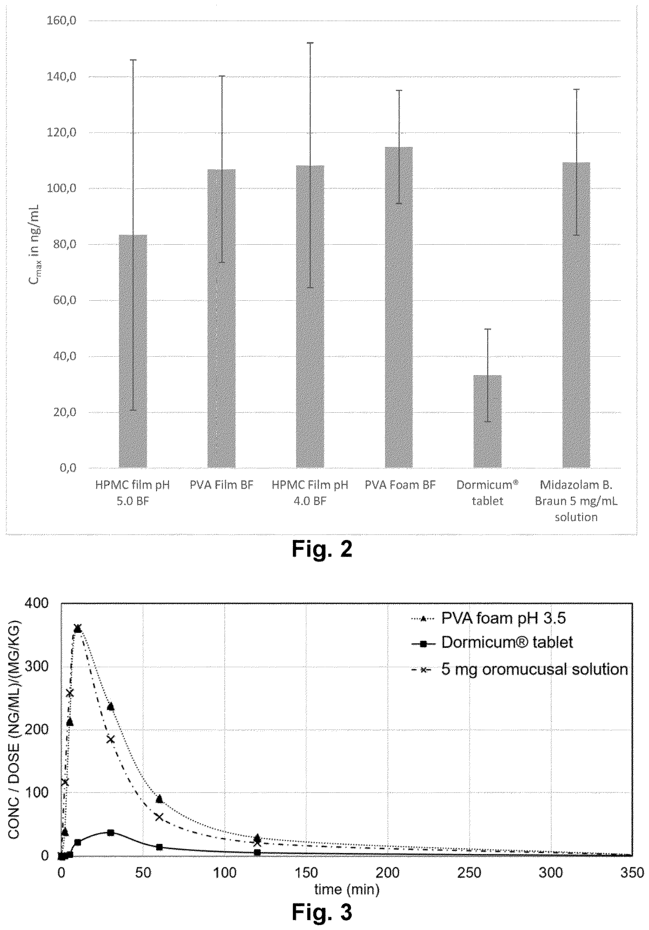

The inventors of the present disclosure found that a cyclodextrin may act as solubility enhancer for midazolam, or a pharmaceutically acceptable salt thereof by encapsulating at least parts of the drug molecule in its inner, lipophilic core, and thus reduces also bitterness of its taste. Surprisingly, it was found that said encapsulation of midazolam or a pharmaceutically acceptable salt thereof does neither reduce (see Figure 2) nor delay (see Figure 1) the absorption of the drug through the oral mucosa. To the contrary, midazolam or a pharmaceutically acceptable salt thereof is rapidly absorbed within about 10 min and taken up into the systemic circulation where it can act pharmacologically as shown in Figures 2 to 6. In other words, midazolam or a pharmaceutically acceptable salt thereof is rapidly released from the cyclodextrin at the oral mucosa. Therefore, the onset of pharmacological action is fast as shown in Figure 1. Without wishing to be bound by theory, it is believed that the drug is directly absorbed at the oral mucosa after release from the cyclodextrin without being solubilized in saliva. In other words, the cyclodextrin directly carries the midazolam or pharmaceutically acceptable salt thereof from the unit dosage composition to the place of absorption.

According to a further preferred embodiment of the present disclosure, the unit dosage composition comprises midazolam hydrochloride and hydroxypropyl-beta-cyclodextrin.

According to another preferred embodiment of the present disclosure, the mass ratio between midazolam free base and hydroxypropyl-beta-cyclodextrin is in the range of from about 1:1 to about 1:10. In other words, the unit dosage composition according to the first aspect of the present disclosure comprises hydroxypropyl-beta-cyclodextrin in a mass that is about one to ten times the mass of midazolam free base. The mass ratio between midazolam free base and hydroxypropyl-beta-cyclodextrin is independent of the counter ion of midazolam, such as the chloride anion in midazolam hydrochloride.

According to another preferred embodiment of the present disclosure, the mass ratio between midazolam free base and hydroxypropyl-beta-cyclodextrin is in the range of from about 1:2 to about 1:8. According to another preferred embodiment of the present disclosure, the mass ratio between midazolam free base and hydroxypropyl-beta-cyclodextrin is in the range of from about 1:3 to about 1:6. According to another preferred embodiment of the present disclosure, the mass ratio between midazolam free base and hydroxypropyl-beta-cyclodextrin is in the range of from about 1:4 to about 1:5.

According to a further preferred embodiment of the present disclosure, the mass ratio between midazolam free base and hydroxypropyl-beta-cyclodextrin is about 1:5.2. In other words, the unit dosage composition according to the first aspect of the present disclosure comprises hydroxypropyl-beta-cyclodextrin in a mass that is about 5.2 times the mass of midazolam free base.

It is understood that the above given ratios of midazolam free base to cyclodextrin may be transferred or recalculated to corresponding salts of midazolam, or to other cyclodextrins according to their respective mass, i.e., molecular weight.

According to another preferred embodiment of the present disclosure, the unit dosage composition comprises a cyclodextrin in an amount of about 0.1 mg or more. According to another preferred embodiment of the present disclosure, the unit dosage composition comprises a cyclodextrin in an amount of about 0.5 mg or more. According to another preferred embodiment of the present disclosure, the unit dosage composition comprises a cyclodextrin in an amount of about 1 mg or more. According to another preferred embodiment of the present disclosure, the unit dosage composition comprises a cyclodextrin in an amount of about 2 mg or more. According to another preferred embodiment of the present disclosure, the unit dosage composition comprises a cyclodextrin in an amount of about 4 mg or more. According to another preferred embodiment of the present disclosure, the unit dosage composition comprises a cyclodextrin in an amount of about 6 mg or more. According to another preferred embodiment of the present disclosure, the unit dosage composition comprises a cyclodextrin in an amount of about 8 mg or more. According to another preferred embodiment of the present disclosure, the unit dosage composition comprises a cyclodextrin in an amount of about 10 mg or more. According to a further preferred embodiment of the present disclosure, the unit dosage composition comprises a cyclodextrin in an amount of about 12 mg or more.

According to a preferred embodiment of the present disclosure, the unit dosage composition comprises a cyclodextrin in an amount of about 150 mg or less. According to another preferred embodiment of the present disclosure, the unit dosage composition comprises a cyclodextrin in an amount of about 120 mg or less. According to another preferred embodiment of the present disclosure, the unit dosage composition comprises a cyclodextrin in an amount of about 100 mg or less. According to another preferred embodiment of the present disclosure, the unit dosage composition comprises a cyclodextrin in an amount of about 80 mg or less. According to another preferred embodiment of the present disclosure, the unit dosage composition comprises a cyclodextrin in an amount of about 60 mg or less. According to another preferred embodiment of the present disclosure, the unit dosage composition comprises a cyclodextrin in an amount of about 50 mg or less. According to another preferred embodiment of the present disclosure, the unit dosage composition comprises a cyclodextrin in an amount of about 40 mg or less. According to another preferred embodiment of the present disclosure, the unit dosage composition comprises a cyclodextrin in an amount of about 30 mg or less. According to another preferred embodiment of the present disclosure, the unit dosage composition comprises a cyclodextrin in an amount of about 25 mg or less. According to a further preferred embodiment of the present disclosure, the unit dosage composition comprises a cyclodextrin in an amount of about 20 mg or less.

According to a preferred embodiment of the present disclosure, the unit dosage composition comprises a cyclodextrin in an amount in the range of from about 0.1 mg to about 150 mg. According to another preferred embodiment of the present disclosure, the unit dosage composition comprises a cyclodextrin in an amount in the range of from about 0.5 mg to about 120 mg. According to another preferred embodiment of the present disclosure, the unit dosage composition comprises a cyclodextrin in an amount in the range of from about 1 mg to about 100 mg. According to another preferred embodiment of the present disclosure, the unit dosage composition comprises a cyclodextrin in an amount in the range of from about 2.0 mg to about 80 mg. According to another preferred embodiment of the present disclosure, the unit dosage composition comprises a cyclodextrin in an amount in the range of from about 4.0 mg to about 60 mg. According to another preferred embodiment of the present disclosure, the unit dosage composition comprises a cyclodextrin in an amount in the range of from about 6.0 mg to about 40 mg. According to another preferred embodiment of the present disclosure, the unit dosage composition comprises a cyclodextrin in an amount in the range of from about 8.0 mg to about 30 mg. According to a further preferred embodiment of the present disclosure, the unit dosage composition comprises a cyclodextrin in an amount in the range of from about 10 mg to about 25 mg. According to a further preferred embodiment of the present disclosure, the unit dosage composition comprises a cyclodextrin in an amount in the range of from about 12 mg to about 20 mg.

It can be concluded from examples 2 and 3 of the present disclosure that the presence of cyclodextrin in the unit dosage compositions of the present disclosure and the encapsulation of midazolam does surprisingly not negatively affect the release of midazolam from the unit dosage composition. The quick release of midazolam from the dosage form is supported by the high bioavailability of midazolam in animal studies as evidenced in example 3 and figures 2 to 6. In other words, the cyclodextrin in the buccal films did not have any negative effect on drug absorption via the oral mucosa, which could have been expected as the drug has to be released from the cyclodextrin complex prior to absorption.

Generally, cyclodextrins are too hydrophilic and bulky to be absorbed significantly via passive diffusion through the oral mucosa. For example, the per-oral bioavailability of hydroxypropyl-beta-cyclodextrin in humans has been reported to be in the range of from about 0.5 to about 3.3 %.

*The matrix material*

According to the first aspect of the present disclosure, the unit dosage composition comprises at least one matrix material.

It is understood that the at least one matrix material acts as a film-forming agent and determines the dissolution characteristics of the oral thin film.

According to a preferred embodiment of the present disclosure, the at least one matrix material is selected from the group consisting of cellulose, cellulose derivatives, acrylic polymer, acrylic co-polymer, and mixtures thereof.

According to a preferred embodiment of the present disclosure, the at least matrix material is selected from the group consisting of ethylcellulose, methylcellulose, hydroxypropyl methylcellulose (HPMC), hydroxyethyl cellulose, hydroxypropyl cellulose, carboxymethylcellulose, carboxymethylcellulose sodium salt, microcrystalline cellulose, croscarmellose, cellulose acetate, polyvinylpyrrolidone, polyvinylalcohol (PVA), polyethylene oxide and mixtures thereof.

According to another preferred embodiment of the present disclosure, the at least matrix material is selected from the group consisting of methylcellulose, hydroxypropyl methylcellulose (HPMC), hydroxyethyl cellulose, hydroxypropyl cellulose, carboxymethylcellulose, carboxymethylcellulose sodium salt, polyvinylpyrrolidone, polyvinylalcohol (PVA), and mixtures thereof.

According to a further preferred embodiment of the present disclosure, the at least matrix material is selected from the group consisting of hydroxypropyl methyl cellulose, polyvinylalcohol (PVA), and mixtures thereof.

According to a further preferred embodiment of the present disclosure, the at least matrix material is hydroxypropyl methylcellulose (HMPC). According to a further preferred embodiment of the present disclosure, the at least matrix material is polyvinylalcohol (PVA).

It is understood that the at least one matrix material provides for a fast disintegration of the oral thin film upon contact with water or saliva as shown in tables 3 to 5. Furthermore, the at least one matrix material provides for an adherence of the oral thin film to the buccal mucosa.

According to a preferred embodiment of the present disclosure, the unit dosage composition comprises the at least one matrix material in an amount of about 5 mg or more. According to another preferred embodiment of the present disclosure, the unit dosage composition comprises the at least one matrix material in an amount of about 10 mg or more. According to another preferred embodiment of the present disclosure, the unit dosage composition comprises the at least one matrix material in an amount of about 15 mg or more. According to a further preferred embodiment of the present disclosure, the unit dosage composition comprises the at least one matrix material in an amount of about 20 mg or more.

According to a preferred embodiment of the present disclosure, the unit dosage composition comprises the at least one matrix material in an amount of about 200 mg or less. According to another preferred embodiment of the present disclosure, the unit dosage composition comprises the at least one matrix material in an amount of about 150 mg or less. According to another preferred embodiment of the present disclosure, the unit dosage composition comprises the at least one matrix material in an amount of about 100 mg or less. According to another preferred embodiment of the present disclosure, the unit dosage composition comprises the at least one matrix material in an amount of about 80 mg or less. According to another preferred embodiment of the present disclosure, the unit dosage composition comprises the at least one matrix material in an amount of about 60 mg or less. According to another preferred embodiment of the present disclosure, the unit dosage composition comprises the at least one matrix material in an amount of about 50 mg or less. According to another preferred embodiment of the present disclosure, the unit dosage composition comprises the at least one matrix material in an amount of about 40 mg or less. According to a further preferred embodiment of the present disclosure, the unit dosage composition comprises the at least one matrix material in an amount of about 35 mg or less.

According to a preferred embodiment of the present disclosure, the unit dosage composition comprises the at least one matrix material in an amount in the range of from about 5 mg to about 200 mg. According to another preferred embodiment of the present disclosure, the unit dosage composition comprises the at least one matrix material in an amount in the range of from about 5 mg to about 150 mg. According to another preferred embodiment of the present disclosure, the unit dosage composition comprises the at least one matrix material in an amount in the range of from about 5 mg to about 100 mg. According to another preferred embodiment of the present disclosure, the unit dosage composition comprises the at least one matrix material in an amount in the range of from about 5 mg to about 60 mg. According to another preferred embodiment of the present disclosure, the unit dosage composition comprises the at least one matrix material in an amount in the range of from about 10 mg to about 50 mg. According to another preferred embodiment of the present disclosure, the unit dosage composition comprises the at least one matrix material in an amount in the range of from about 15 mg to about 40 mg. According to another preferred embodiment of the present disclosure, the unit dosage composition comprises the at least one matrix material in an amount in the range of from about 15 mg to about 35 mg. According to a further preferred embodiment of the present disclosure, the unit dosage composition comprises the at least one matrix material in an amount in the range of from about 20 mg to about 35 mg.

According to a preferred embodiment, the at least one matrix material is a mixture of two matrix materials with different viscosities. This allows for the adjustment of the viscosity of the solution used to manufacture the final oral thin film and the film itself.

According to a preferred embodiment, the at least one matrix material is a mixture of two matrix materials with different viscosities selected from the same chemical type of material.

According to another preferred embodiment of the present disclosure, the mixture of two matrix materials consists of a first matrix material and a second matrix material, wherein the first matrix material has an about 1.2-fold to about 100-fold higher viscosity than the second matrix material. According to another preferred embodiment of the present disclosure, the mixture of two matrix materials consists of a first matrix material and a second matrix material, wherein the first matrix material has an about 2-fold to about 50-fold higher viscosity than the second matrix material. According to another preferred embodiment of the present disclosure, the mixture of two matrix materials consists of a first matrix material and a second matrix material, wherein the first matrix material has an about 5-fold to about 20-fold higher viscosity than the second matrix material.

It is assumed that a mixture of two matrix materials of different viscosities improves the manufacturability of the oral thin films.

*Further components*

According to a preferred embodiment of the first aspect of the present disclosure, the unit dosage composition additionally comprises at least one sweetening agent.

According to another preferred embodiment of the present disclosure, the at least one sweetening agent is selected from the group consisting of sucralose, sodium saccharin, and mixtures thereof.

According to a further preferred embodiment of the present disclosure, the at least one sweetening agent is sucralose. According to a further preferred embodiment of the present disclosure, the at least one sweetening agent is sodium saccharin.

According to a preferred embodiment of the present disclosure, the unit dosage composition comprises the at least one sweetening agent in an amount of about 20 mg or less. According to another preferred embodiment of the present disclosure, the unit dosage composition comprises the at least one sweetening agent in an amount of about 15 mg or less. According to another preferred embodiment of the present disclosure, the unit dosage composition comprises the at least one sweetening agent in an amount of about 10 mg or less.

According to a preferred embodiment of the present disclosure, the unit dosage composition comprises the at least one sweetening agent in an amount of about 1 mg or more. According to another preferred embodiment of the present disclosure, the unit dosage composition comprises the at least one sweetening agent in an amount of about 2 mg or more.

It is understood that the presence of at least one sweetening agent in the unit dosage composition according to the present disclosure further increases the application comfort.

According to another preferred embodiment of the present disclosure, the unit dosage composition of the first aspect of the present disclosure further comprises glycerol.

According to a preferred embodiment of the present disclosure, the unit dosage composition comprises glycerol in an amount of about 20 mg or less. According to another preferred embodiment of the present disclosure, the unit dosage composition comprises glycerol in an amount of about 15 mg or less. According to another preferred embodiment of the present disclosure, the unit dosage composition comprises glycerol in an amount of about 12 mg or less.

According to a preferred embodiment of the present disclosure, the unit dosage composition comprises glycerol in an amount of about 1 mg or more. According to another preferred embodiment of the present disclosure, the unit dosage composition comprises glycerol in an amount of about 3 mg or more. According to another preferred embodiment of the present disclosure, the unit dosage composition comprises glycerol in an amount of about 5 mg or more.

It is understood that the presence of glycerol acts as a plasticizer in the unit dosage composition of the present disclosure. In other words, glycerol makes the oral thin film soft and flexible, and breaking is avoided. The amount of glycerol can be adjusted according to the needs.

According to another preferred embodiment of the present disclosure, the unit dosage composition has a pH in the range of from about 2.0 to about 8.0. According to another preferred embodiment of the present disclosure, the unit dosage composition has a pH in the range of from about 2.5 to about 7.0. According to another preferred embodiment of the present disclosure, the unit dosage composition has a pH in the range of from about 3.0 to about 6.0. According to a further preferred embodiment of the present disclosure, the unit dosage composition has a pH in the range of from about 3.5 to about 5.0.

The pH of the unit dosage composition can be adjusted during manufacture by use of any pharmaceutically acceptable acid or base. It is preferable that the pH is adjusted with citric acid.

The method of manufacture

According to a second aspect, the present disclosure relates to a method for manufacturing the unit dosage composition according to the first aspect of the present disclosure, comprising the steps of

a) providing an aqueous solution comprising midazolam or a pharmaceutically acceptable salt thereof, a cyclodextrin, and at least one matrix material,
b) coating the solution an a liner, and
c) allowing the solution to dry and form a film.

According to a preferred embodiment of the present disclosure, the method for manufacturing the unit dosage composition according to the first aspect of the present disclosure further comprises the steps of adjusting the pH and/or foaming the solution prior to coating.

Medical use

According to a third aspect, the present disclosure relates to a unit dosage composition according to the first aspect of the present disclosure for use in the treatment or prevention of febrile seizure, epileptic seizure, such as status epilepticus, fear, such as preoperative anxiolysis to reduce fear, and/or agitation, such as agitation prior to surgery.

According to a preferred embodiment, the present disclosure relates to a unit dosage composition according to the first aspect of the present disclosure for use in the treatment of febrile seizure. According to another preferred embodiment, the present disclosure relates to a unit dosage composition according to the first aspect of the present disclosure for use in the treatment of epileptic seizure, such as status epilepticus. According to a further preferred embodiment, the present disclosure relates to a unit dosage composition according to the first aspect of the present disclosure for use in the treatment of status epilepticus. According to another preferred embodiment, the present disclosure relates to a unit dosage composition according to the first aspect of the present disclosure for use in the treatment of preoperative fear. According to a further preferred embodiment, the present disclosure relates to a unit dosage composition according to the first aspect of the present disclosure for use in a method of preoperative anxiolysis to reduce fear. According to another preferred embodiment, the present disclosure relates to a unit dosage composition according to the first aspect of the present disclosure for use in the treatment of agitation, such as agitation prior to surgery. According to a further preferred embodiment, the present disclosure relates to a unit dosage composition according to the first aspect of the present disclosure for use in the treatment of agitation before surgery.

According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is reached within less than about 30 minutes. According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is reached within less than about 20 minutes. According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is reached within less than about 15 minutes. According to a further preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject, wherein the unit dosage composition is administered into the oral cavity of a subject and the therapeutically effective concentration of midazolam in the blood of said subject is reached within less than about 10 minutes.

According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for more than about 10 minutes and up to about 120 minutes. According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for more than about 20 minutes and up to about 90 minutes. According to a further preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for more than about 30 minutes and less than about 90 minutes.

According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for more than about 10 minutes. According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for more than about 20 minutes. According to a further preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for more than about 30 minutes.

According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for about 120 minutes or less. According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for about 90 minutes or less. According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the therapeutically effective concentration of midazolam in the blood of said subject is maintained for less than about 90 minutes.

According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the half-life of midazolam in the blood of said subject is in the range of from about 10 minutes to about 240 minutes. According to another preferred embodiment, the present disclosure relates to a unit dosage composition according to the first aspect of the present disclosure for use in the treatment according to the third aspect of the present disclosure, wherein the unit dosage composition is applied in the oral mucosa of a subject and the half-life of midazolam in the blood of said subject is in the range of from about 20 minutes to about 120 minutes. According to another preferred embodiment, the present disclosure relates to a unit dosage composition according to the first aspect of the present disclosure for use in the treatment according to the third aspect of the present disclosure, wherein the unit dosage composition is applied in the oral mucosa of a subject and the half-life of midazolam in the blood of said subject is in the range of from about 30 minutes to about 90 minutes. According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the half-life of midazolam in the blood of said subject is in the range of from about 40 minutes to about 60 minutes.

According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the clearance in the blood of said subject is in the range of from about 0.005 L/(min*kg) to about 0.25 L/(min*kg). According to another preferred embodiment, the present disclosure relates to a unit dosage composition according to the first aspect of the present disclosure for use in the treatment according to the third aspect of the present disclosure, wherein the unit dosage composition is administered into the oral cavity of a subject and the clearance in the blood of said subject is in the range of from about 0.01 L/(min*kg) to about 0.2 L/(min*kg). According to another preferred embodiment, the present disclosure relates to a unit dosage composition according to the first aspect of the present disclosure for use in the treatment according to the third aspect of the present disclosure, wherein the unit dosage composition is applied in the oral mucosa of a subject and the clearance in the blood of said subject is in the range of from about 0.02 L/(min*kg) to about 0.15 L/(min*kg).

According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the distribution volume in the blood of said subject is in the range of from about 0.5 L/kg to about 20 L/kg. According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the distribution volume in the blood of said subject is in the range of from about 1 L/kg to about 20 L/kg. According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the distribution volume in the blood of said subject is in the range of from about 2 L/kg to about 10 L/kg.

According to a preferred embodiment, the present disclosure relates to a unit dosage composition according to the first aspect of the present disclosure for use in the treatment according to the third aspect of the present disclosure, wherein the unit dosage composition is administered into the oral cavity of a subject and the profiles of the plasma concentration per dose of said subject are not subjected to a gender effect.

Method of treatment

According to a fourth aspect, the present disclosure relates to a method of treatment or prevention of febrile seizure, epileptic seizure, such as status epilepticus, fear, such as preoperative anxiolysis to reduce fear, and/or agitation, such as agitation prior to surgery, wherein the unit dosage composition according to the first aspect of the present disclosure is administered to a subject.

Use in the manufacture of a medicament

According to a fifth aspect, the present disclosure relates to a use of the unit dosage composition according to the first aspect of the present disclosure in the manufacture of a medicament for the treatment of febrile seizure, epileptic seizure, such as status epilepticus, fear, such as preoperative anxiolysis to reduce fear, and/or agitation, such as agitation prior to surgery.

Definitions and general embodiments

As used herein, the term "oral thin film" that may be abbreviated as OTF refers to a solid dosage form consisting of single- or multilayer sheets of suitable materials and intended for administration in the mouth to deliver active substances. Oral thin films are also herein referred to as "wafer".

As used herein, "intraoral administration" is a topical route of administration comprising buccal or sub lingual administration. For buccal administration drugs held or applied in the buccal area diffusing through the oral mucosa and entering directly into the bloodstream. Typically, the drug is placed between gums and the inner lining of the cheek.

As used herein, the term "midazolam" relates to the free base of midazolam, which is also known as 8-chloro-6-(2-fluoropheny)-1-methyl-4$H$-imidazo[1,5-$\alpha$][1,4]benzodiazepine, or CAS number 59467-70-8. Midazolam has the molecular formula $C_{18}H_{13}ClFN_3$ and has a molecular weight of 325.77 g/mol. The terms "midazolam" and "midazolam free base" are used interchangeably.

As used herein, the term "pharmaceutically acceptable salt of midazolam" refers to a salt of midazolam that retains the desired biological and pharmaceutical activity of midazolam and includes a pharmaceutically acceptable acid addition salt. Suitable pharmaceutically acceptable acid addition salts of midazolam may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic, and arylsulfonic acid.

As used herein, the "hydrochloride salt of midazolam" or "midazolam hydrochloride", which are use synonymously, is known under CAS number 59467-96-8 and is the addition salt of midazolam and hydrochloric acid (HCl).

As used herein, the "maleate salt of midazolam" or "midazolam maleate", which are use synonymously, is known under CAS number 59467-94-6 and is the addition salt of midazolam and maleic acid ($HO_2CCH=CHCO_2H$). This comprises also midazolam hydrogen maleate.

As used herein, the "amount of midazolam" is determined on the basis of midazolam free base, unless specified otherwise. The "amount of midazolam" is also referred to as "m(midazolam)". In other words, the "amount of midazolam" as used herein is the mass of midazolam or a pharmaceutically acceptable salt thereof expressed as the corresponding mass of midazolam free base. Hence, it is understood that the mass of midazolam free base is the "amount of midazolam". For pharmaceutically acceptable salts of midazolam, the "amount of midazolam" is calculated from the mass of the pharmaceutically acceptable salt of midazolam, which is also referred to as "m(midazolam salt)", by formula (I):

$$m(\text{midazolam}) = m(\text{midazolam salt}) \cdot \frac{325.77 \text{ g/mol}}{M(\text{midazolam salt})} \qquad (I)$$

wherein m(midazolam) is the "amount of midazolam", m(midazolam salt) is the mass of the pharmaceutically acceptable salt of midazolam, and M(midazolam salt) is the molecular weight of the respective pharmaceutically acceptable salt of midazolam.

For example, when midazolam hydrochloride is used as pharmaceutically acceptable salt of midazolam in a unit dosage composition according to the present disclosure, the above formula I simplifies as follows with the molecular weight of midazolam hydrochloride M(midazolam hydrochloride) being 362.23 g/mol:

$$m(\text{midazolam}) = m(\text{midazolam hydrochloride}) \cdot \frac{325.77 \frac{g}{mol}}{362.23 \frac{g}{mol}}$$

$$= m(\text{midazolam hydrochloride}) \cdot 0.9$$

wherein m(midazolam) is the "amount of midazolam" (mass), m(midazolam hydrochloride) is the amount of midazolam hydrochloride (mass).

For example, when midazolam maleate is used as pharmaceutically acceptable salt of midazolam in a unit dosage composition according to the present disclosure, the above formula I simplifies as follows with the molecular weight of midazolam maleate M(midazolam maleate) being 441.84 g/mol:

$$m(\text{midazolam}) = m(\text{midazolam maleate}) \cdot \frac{325.77 \frac{g}{mol}}{441.84 \frac{g}{mol}} = m(\text{midazolam maleate}) \cdot 0.74$$

wherein m(midazolam) is the "amount of midazolam" (mass), m(midazolam maleate) is the amount of midazolam maleate (mass).

As used herein, the term "cyclodextrin" refers to a cyclic oligosaccharide consisting of a macrocyclic ring of glucose subunits connected by $\alpha$-1,4-glycosidic bonds, and derivatives thereof.

As used herein, the terms "alpha-cyclodextrin" and "a-cyclodextrin" are used interchangeably and refer to a cyclodextrin that is a cyclic oligosaccharide consisting of a macrocyclic ring of six glucose subunits connected by $\alpha$-1,4-glycosidic bonds, and derivatives thereof. Alpha-cyclodextrin is also known under its CAS-number 10016-20-3.

As used herein, the terms "beta-cyclodextrin" and "$\beta$-cyclodextrin" are used interchangeably and refer to a cyclodextrin that is a cyclic oligosaccharide consisting of a macrocyclic ring of seven glucose subunits connected by $\alpha$-1,4-glycosidic bonds, and derivatives thereof. Beta-cyclodextrin is also known under its CAS-number 7585-39-9.

As used herein, the terms "gamma-cyclodextrin" and "y-cyclodextrin" are used interchangeably and refer to a cyclodextrin that is a cyclic oligosaccharide consisting of a macrocyclic ring of eight glucose subunits connected by $\alpha$-1,4-glycosidic bonds, and derivatives thereof. Gamma-cyclodextrin is also known under its CAS-number 17465-86-0.

As used herein, the terms "derivative of cyclodextrin" and "cyclodextrin derivative" are used interchangeably and refer to a cyclodextrin in which the hydrogen of at least one OH-group in each of the glucose subunits contained in the cyclodextrin is replaced with an unsubstituted or substituted $(C_1-C_{10})$alkyl. According to a preferred embodiment of the present disclosure, the hydrogen of one OH-group, two OH-groups, or three OH-groups in each glucose subunit contained in the cyclodextrin is replaced with an unsubstituted or substituted $(C_1-C_{10})$alkyl.

If the hydrogen of one OH-group in each glucose subunit contained in the cyclodextrin is replaced with an unsubstituted or substituted $(C_1-C_{10})$alkyl, it is preferred that the hydrogen of the OH-group on $C_6$ of each of the glucose subunits contained in the cyclodextrin is replaced with an unsubstituted or substituted $(C_1-C_{10})$alkyl.

For the purpose of the present disclosure, the term "$(C_1-C_{10})$alkyl" as used by itself or as part of another group refers to a straight- or branched-chain aliphatic hydrocarbon containing one to ten, i.e. 1, 2, 3, 4, 5, 6, 7, 8, 9, or 10, carbon atoms (i.e., $C_1-C_{10}$ alkyl) or the number of carbon atoms designated (i.e., a $C_1$ alkyl such as methyl, a $C_2$ alkyl such as ethyl, a $C_3$ alkyl such as propyl or isopropyl, etc.). In one embodiment, the alkyl group is chosen from a straight

chain $(C_1-C_{10})$alkyl group. In another embodiment, the alkyl group is chosen from a branched chain $(C_3-C_{10})$alkyl group. In another embodiment, the alkyl group is chosen from a straight chain $(C_1-C_6)$alkyl group. In another embodiment, the alkyl group is chosen from a branched chain $(C_3-C_6)$alkyl group. In another embodiment, the alkyl group is chosen from a straight chain $(C_1-C_4)$alkyl group. In another embodiment, the alkyl group is chosen from a branched chain $(C_3-C_4)$alkyl group. In another embodiment, the alkyl group is chosen from a straight or branched chain $(C_3-C_4)$alkyl group. Non-limiting exemplary $(C_1-C_{10})$alkyl groups include methyl, ethyl, propyl, isopropyl, butyl, sec-butyl, tert-butyl, iso-butyl, 3-pentyl, hexyl, heptyl, octyl, nonyl, decyl, and the like. Non-limiting exemplary $C_{1-4}$ alkyl groups include methyl, ethyl, propyl, isopropyl, butyl, sec-butyl, tert-butyl, and iso-butyl.

As used herein, the term "unsubstituted" means that there is no substituent or that the only substituents are hydrogen.

The term "substituted" as used throughout the specification denotes that the group contains one or more substituent groups. Preferably the substituent groups are one or more groups independently selected from the group consisting of halogen, =O, =S, -CN, -NOz, - $CF_3$, -$OCF_3$, and -OH. Non-limiting exemplary substituted $(C_1-C_{10})$alkyl groups include -$CH_2CH_2NO_2$, -$CH_2CH_2OH$, -$CH_2CH(OH)CH_3$, -$CH_2CH_2CH_2OH$, and the like.

For the purpose of the present disclosure, the term "substituted $(C_1-C_{10})$alkyl" preferably refers to a "$(C_1-C_{10})$hydroxyalkyl" that refers to a $(C_1-C_{10})$alkyl group substituted with one or more, e.g., one, two, or three, hydroxy groups. In one embodiment, the $(C_1-C_{10})$hydroxyalkyl group is a monohydroxy-$(C_1-C_{10})$alkyl group, *i.e.,* substituted with one hydroxy group. In another embodiment, the $(C_1-C_{10})$hydroxyalkyl group is a dihydroxy-$(C_1-C_{10})$alkyl group, *i.e.,* substituted with two hydroxy groups. In another embodiment, the hydroxyalkyl group is chosen from a $(C_1-C_4)$hydroxyalkyl group. Non-limiting exemplary $(C_1-C_{10})$hydroxyalkyl groups include hydroxyethyl, hydroxypropyl and hydroxybutyl groups, such as 1-hydroxyethyl, 2-hydroxyethyl, 1,2-dihydroxyethyl, 2-hydroxypropyl, 3-hydroxypropyl, 3-hydroxybutyl, 4-hydroxybutyl, 2-hydroxy-1-methylpropyl, and 1,3-dihydroxyprop-2-yl.

Non-limiting examples of "cyclodextrin derivative" are permethyl-beta-cyclodextrin, and hydroxypropyl-beta-cyclodextrin.

As used herein, "permethyl-beta-cyclodextrin" refers to a beta-cyclodextrin in which each hydrogen of each of the OH-groups in the beta-cyclodextrin is replaced by -$CH_3$. Permethyl-beta-cyclodextrin is also known under CAS-number 55216-11-0.

As used herein, "Hydroxypropyl-beta-cyclodextrin", that can be abbreviated as "HPbCD", refers to a beta-cyclodextrin in which each hydrogen of each the OH-groups on $C_6$ of each glucose subunit contained in the beta-cyclodextrin is replaced by -$CH_2CH(OH)CH_3$. Hydroxypropyl-beta-cyclodextrin is also known under CAS-number 128446-35-5.

As used herein, "hydroxypropyl methylcellulose", that can be abbreviated as "HPMC", refers to a semisynthetic, inert, viscoelastic polymer that is also known under its CAS number 9004-65-3 or as E464.

As used herein, "polyvinyl alcohol", that can be abbreviated as "PVA", refers to a synthetic, water-soluble polymer that is also known under its CAS number 9002-89-5 or as E1203.

As used herein, the "viscosity" is measured by a viscometer.

As used herein, the "amount of midazolam, or a pharmaceutically acceptable salt thereof" is measured by reversed phase HPLC analysis combined with UV detection. For the mobile phase, a solution of 7.7 g/L ammonium acetate and 10 mUL tetrabutly ammoniumhydroxide solution 40 % (400 g/L) was prepared, pH was adjusted to 5,3 with acetic acid. 49.5 volume parts of this solution were mixed with 50.5 volume parts of methanol, constituting the mobile phase. For the analysis, the waver was dissolved in 10 mL of mobile phase by stirring for at least 30 min in a closed container until the waver is completely dissolved (visual control). The solution was then subjected to HPLC on a column "Phenomenex Luna C8(2) 100A, 150 × 4,6 mm, 3 µm, with a flow rate of 1.0 µL/min. Midazolam was detected at a wavelength of 254 nm after about 19 min.

As used herein, the "water content" is measured by Karl-Fischer titration and coulorimetric quantification in a gas extraction method using Karl-Fischer coulometer and a Karl-Fischer oven. The weight of an empty ampule with crimp cap is taken, a waver is put into the ampule and the ampule is sealed gas-tight with the crimp cap, and the weight is determined again. The water is extracted at 120 °C in a Karl-Fischer oven.

As used herein, the "disintegration time" is measured by a modified USP <701> disintegration test. The time until a waver in aqueous medium is torn apart is determined. The waver is provided at the lower end with a metallic clip, while the upper end is clipped to the basket. The basket is put into the medium (800 mL of deionized and degassed water at 37 ±2 °C), and if the waver is torn apart, the clip is sinking to the bottom of the basket, and the measurement is stopped automatically.

Where the term "comprising" is used in the present description and claims, it does not exclude other elements. For the purposes of the present disclosure, the term "consisting of" is considered to be a preferred embodiment of the term "comprising". If a group is defined to comprise at least a certain number of embodiments herein, this is also to be understood to disclose a group, which preferably consists only of these embodiments. Furthermore, if a composition is defined using the term "comprising", it may additionally comprise other elements not explicitly listed, however, not further amounts of an element listed. As such, if, e.g., unit dosage composition comprises midazolam, or a pharmaceutically acceptable salt thereof in an amount of 2.5 mg, said unit dosage composition may comprise elements other than midazolam, or a pharmaceutically acceptable salt thereof, however, not additional amounts of midazolam, or a pharmaceutically acceptable salt thereof, thereby exceeding the amount of 2.5 mg.

The term "about" in conjunction with a numerical value refers to normal deviations of said numerical value. It is to be understood that the term "about" can mean a deviation of $\pm$ 10 %, preferably $\pm$ 5 %, more preferably $\pm$ 2.5 % of said numeric value as indicated.

**Examples**

Example 1: Manufacture of oral thin films

Unless specified otherwise, all chemicals used herein were obtained from commercial suppliers. PVA 4-88, and PVA 40-88 were obtained from Merck KGaA (Germany). Polyvinyl alcohol (PVA) 4-88 was purchased as Mowiol 4-88, having a viscosity (40 g/L in water) of 3.4-4.6 mPa·s (goal: 4 mPa s), and PVA 40-88 having a viscosity of 34-46 mPa·s (goal: 40 mPa s). HPMC 606 and HPMC 60SH50 (both hydroxypropyl methyl cellulose 2910) were purchased as Pharmacoat 606 and Metolose 60SH-50, respectively, from Shin-Etsu Chemical Co. Ltd. Viscosity of HPMC grades was measured in aqueous solution at 2 % by weight at 20 °C according to USP method (Hypromellose), resulting in 4.8-7.2 mPa·s (goal: 6 mPa s) for HPMC 606, and 40-60 mPa·s (goal: 50 mPa s) for HPMC 60SH50.

For the manufacture of oral thin films, a coating mass was prepared by dissolving the components of the oral thin film to be prepared in water while stirring at 300 to 800 rpm until a clear and homogenous solution of the composition as shown in Table 1 was formed. For the oral thin film "PVA foam pH 3.5", the stirring speed was increased to 1400 rpm for foaming. The pH of the solutions was adjusted with small amounts of citric acid. During the adjustment of the HPMC film to pH 5.0, parts of the midazolam reprecipitated creating a homogenous whitish suspension which was processed in the following steps.

**Table 1** Composition of oral thin films

| oral thin film | PVA foam pH 3.5 | | PVA film pH 3.5 | | HPMC film pH 4.0 | | HPMC film pH 5.0 | |
|---|---|---|---|---|---|---|---|---|
| | solution | dried | sol. | dried | sol. | dried | sol. | dried |
| | [wt.-%] | | [wt.-%] | | [wt.-%] | | [wt.-%] | |
| Midazolam HCl | 1.70 | 5.33 | 1.97 | 4.92 | 1.92 | 6.00 | 1.92 | 6.00 |
| PVA 4-88 (35 wt.-% solution) | 44.20 | 48.34 | 49.69 | 43.4 8 | - | - | - | - |
| PVA 40-88 (15 wt.-% solution) | 11.50 | 5.39 | 15.12 | 5.67 | - | - | - | - |
| HPMC 606 | - | - | - | | 10.3 8 | 32.43 | 10.38 | 32.43 |
| HPMC 60SH50 | - | - | - | | 2.76 | 8.61 | 2.76 | 8.61 |
| Glycerol | 3.51 | 10.96 | 7.15 | 17.8 7 | 4.80 | 15.00 | 4.80 | 15.00 |
| HpbCD | 7.96 | 24.89 | 9.19 | 22.9 7 | 8.96 | 28.00 | 8.96 | 28.00 |
| sucralose | 1.63 | 5.09 | 2.04 | 5.09 | 2.12 | 6.64 | 2.12 | 6.64 |
| Na saccharin | - | - | - | - | 1.06 | 3.33 | 1.06 | 3.33 |
| purified water | 29.50 | - | 14.85 | - | 68.0 0 | - | 68.00 | - |
| citric acid | q.s. | | q.s. | | q.s. | | q.s. | |

The solutions were optionally stored for 1 to 2 days for degassing, e.g., for HPMC based films.

Then, the solutions were coated onto a process liner with a 500 μm coating gap through casting and placed in a drying oven for 10-15 min at 30 °C. Afterwards, the temperature was increased to 60 °C over the course of 10-15 min and optionally up to another 15 min at 60 °C until a soft film formed.

Oral thin films for testing were punched with a multiple die-cut to yield square-shaped oral thin films with a size of 5 cm$^2$, pouched, sealed, and labeled.

The oral thin films "PVA foam pH 3.5", "HPMC film pH 4.0", and "HPMC film pH 5.0" thus obtained were stable, not brittle or sticky.

The amount of midazolam in each of the oral thin films was determined according to the procedure outlined above.

Table 2 Content of Midazolam free base in the oral thin films

|  | PVA foam pH 3.5 | PVA film pH 3.5 | HPMC film pH 4.0 | HPMC film pH 5.0 |
|---|---|---|---|---|
| Midazolam free base [mg] | 2.9 | 2.9 | 2.8 | 2.7 |

Example 2: Stability tests

The oral thin films prepared in example 1 were stored at 40 °C and 75 % relative humidity. Samples for stability tests were drawn after 1.5, 3, and 6-months of storage and analyzed for their appearance, water content, content of midazolam, and disintegration time.

The results of the stability tests are summarized in Table 3 to Table 5.

Table 3 Results of stability tests of oral thin film "PVA foam pH 3.5"

| storage time [months] | 0 | 1.5 | 3 | 6 |
|---|---|---|---|---|
| Midazolam free base [% of nominal content] | 106.7 | 108.6 | 104.9 | 105.3 |
| Water content [wt.-%] | 3.8 | n.d. | 4.3 | 4.0 |
| disintegration time [s] | 9/12/8 | n.d. | 12/12/9 | 10/13/11 |
| n.d. = not determined | | | | |

Table 4 Results of stability tests of oral thin film "PVA film pH 3.5"

| storage time [months] | 0 | 1.5 | 3 | 6 |
|---|---|---|---|---|
| Midazolam free base [% of nominal content] | 106.8 | 107.6 | 101.9 | 105.7 |
| Water content [wt.-%] | 4.9 | n.d. | 5.4 | 5.5 |
| disintegration time [s] | 13/16/14 | n.d. | 16/15/16 | 15/15/15 |
| n.d. = not determined | | | | |

Table 5 Results of stability tests of oral thin film "HPMC film pH 4.0"

| storage time [months] | 0 | 1.5 | 3 | 6 |
|---|---|---|---|---|
| Midazolam free base [% of nominal content] | 103.3 | 102.7 | 99.5 | 101.1 |
| Water content [wt.-%] | 6.0 | | 6.9 | 6.6 |
| disintegration time [s] | 10/19/19 | n.d. | 9/9/10 | 23/10/10 |
| n.d. = not determined | | | | |

The films did not show any visible changes in their appearance over time. Moreover there was no indication for recrystallization of the active ingredient, confirmed by XRPD measurements.

The water content of the films, amount of midazolam, and the *in vitro* disintegration time did not change significantly over the storage period of 6 months.

Furthermore, for all oral thin films that have been stored for 3 months at 40 °C / 75 % RH, more than 80 % of drug were released within 3 min.

In conclusions, all oral thin films are considered sufficiently stable to be introduced into preclinical studies.

Example 3: Pharmacokinetic evaluation of oral thin films

To investigate the local tolerability and pharmacokinetics of the four oral thin films prepared according to Example 1, the oral thin films containing 3.0 mg of midazolam HCl (corresponding to 2.7 mg of midazolam free base) were subsequently applied to 6 non-naïve Marshall Beagle dogs (3 male weighing 8.6-11 kg and 3 female weighing 7.5-8.1 kg) with at least 3 days washout phase between the treatments. Dormicum® 7.5 mg tablets and a Midazolam 5 mg/mL solution for injection/infusion (obtained from B. Braun) were used as reference. The dogs were fastened before dose administration and during the period of the experiment. On the days of treatment, the animals were offered their first daily meal after collection of the blood sample 2 hours after dose, and the second meal of the day was given after collection of the blood sample 6 hours after dose.

No oral tissue reaction at the application site was observed.

Blood samples were taken from the dogs before the treatment, and 2, 5, 10, 30, 60, 120, and 360 minutes after the dose administration. Blood samples of approximately 2 mL were collected from the saphenous vein. The blood was sampled into vacutainers containing Lithium Heparin as anticoagulant. The vacutainer was placed in ice water until centrifugation (10 min, 1270 G, +4 °C). Each plasma sample was divided into two aliquots of at least 0.4 mL and transferred to Nunc cryotubes (polypropylene tubes with internal thread) and frozen at -18 °C or below within 90 minutes after collection.

The concentration of midazolam in the blood samples was determined using validated liquid chromatography-mass spectrometry (LC-MS/MS) methods. The calibration range was of from 0.1-1000 ng/mL. In brief, each analytical batch sequence consisted of plasma calibrators including blank and zero samples covering the assay range of from 0.100 ng/mL to 1000 ng/mL for the matrix. Study samples were assayed in profiles per animal. The method performance was assured by co-assaying quality control (QC) samples in duplicate at three concentration levels (0.5, 250 and 800 ng/mL) in matrix. The concentration of analytes in unknown samples was calculated using the analyte/internal standard area ratios. Linear regression was applied for the construction of the calibration curve and a weighting of $1/x2$. All instrument control, data collection, peak area integration and storage was performed using Analyst ver. 1.7.2 (AB Sciex). Study samples were prepared by precipitating an aliquot of 40 $\mu$l plasma plus 5 $\mu$l IS with 200 $\mu$l acetonitrile. It was well mixed, centrifuged for 10 min at 8 °C at 14000 g. The supernatant (190 $\mu$l) was transferred to an HPLC vial. 190 $\mu$l of Mobile Phase - Eluent A was added. It was well mixed and the samples analyzed by using LC-MS/MS.

**Table 6** The HPLC conditions were:

| Analytical Column | Kinetex XB-C18 (2.6$\mu$m 50×2.1mm) |
|---|---|
| Column temperature | 35 °C |
| Mobile Phase | Eluent A: Water + 0.05% formic acid (v/v) Eluent B: Methanol + 0.05% formic acid (v/v) |
| Injection volume | 2 $\mu$l |
| Autosampler temperature | 15 °C |
| Detector | MS/MS |

**Table 7** Gradient programme:

| Time (min) | Flow | %A |
|---|---|---|
| 0.0 | 700 | 97 |
| 1.0 | 700 | 85 |
| 4.5 | 700 | 55 |
| 5.0 | 700 | 8 |
| 5.5 | 700 | 8 |
| 6.0 | 700 | 97 |
| 10 | 700 | 97 |

**Table 8** Valve programme:

| Time(min) | Position |
|-----------|----------|
| 0.0 | Waste |
| 2.5 | MS |
| 5.5 | Waste |

**Table 9** The Mass spectrometer conditions were:

| Mass Spectrometer | Sciex API 6500+ triple quadropole |
|-------------------|-----------------------------------|
| Ionisation mode | Turbo V® Ionspray +ve ion |
| Temperature | 750 °C |
| Curtain Gas | 40 |
| Nebulizer Gas | 60 |
| Heater Gas | 50 |
| Collision Gas | 9.0 (nitrogen) |
| Ion (MRM) transitions monitored | Midazolam $m/z$ 326.0 → 291.1 (dwell 100 ms, CE 38) ISTD $m/z$ 330.0 → 295.0 (dwell 75 ms, CE 38) |

The results of the pharmacokinetic analysis are summarized in Table 10 and Figures 1 to 6.

The maximum exposure ($T_{max}$) occurred faster for all buccal films compared to the reference tablet (10 min vs. 30 min) while the half-life was similar for the tablet and buccal formulations. Furthermore, the buccal films of example 1 showed the same or highly similar maximum plasma concentration of midazolam ($C_{max}$) as the reference solution. $C_{max}$ and $AUC_{inf}$ of all buccal films was considerably higher than for the comparative Dormicum® tablet. The clearance was higher for the Dormicum® tablet than for all the buccal film formulations.

No significant difference in pharmacokinetic profiles was observed between the four buccal film formulations.

**Table 10** Results of pharmacokinetic studies: Mean pharmacokinetic parameters of Midazolam in plasma. Mean plasma pharmacokinetic parameters from individual profiles after buccal or oral administration of Midazolam to dogs.

| Treatment | Gender | $AUC_{inf}$ [min*ng/mL] | $AUC_{inf}/D$ [(min*ng/mL)/ (mg/kg)] | $AUC_{last}$ [min*ng/mL] | $C_{max}$ [ng/mL] | $C_{max}/D$ [(ng/mL)/ (mg/kg)] | $T_{max}$ [min] | $V_zF$ [L/kg] | CLF [L/min/kg] | $T_{1/2}$ [min] |
|---|---|---|---|---|---|---|---|---|---|---|
| PVA Foam pH 3.5 | male | 5888 | 22057 | 5865 | 120 | 448 | 10 | 3.27 | 0.0506 | 46 |
| | female | 6328 | 18052 | 6307 | 110 | 319 | 17 | 3.64 | 0.0569 | 45 |
| PVA Film pH 3.5 | male | 6242 | 23019 | 6221 | 119 | 441 | 10 | 2.91 | 0.0436 | 46 |
| | female | 4841 | 14222 | 4817 | 95 | 278 | 10 | 5.64 | 0.0779 | 51 |
| HPMC Film pH 4.0 | male | 6656 | 24086 | 6633 | 134 | 484 | 10 | 2.90 | 0.0432 | 46 |
| | female | 4572 | 13317 | 4549 | 83 | 242 | 10 | 6.44 | 0.0839 | 52 |
| HPMC Film pH 5.0 | male | 3689 | 12807 | 3680 | 72 | 251 | 17 | 8.54 | 0.1142 | 49 |
| | female | 5051 | 14163 | 5032 | 94 | 264 | 10 | 9.15 | 0.1178 | 50 |
| Dormicum® 7.5 mg tablet (Reference) | male | 2120 | 3090 | 1906 | 27 | 39 | 33 | 26.92 | 0.3273 | 57 |
| | female | 2687 | 2693 | 2651 | 39 | 40 | 23 | 48.97 | 0.4486 | 67 |
| Midazolam 5 mg/mL solution (Reference) | male | 4829 | 18773 | 4804 | 112 | 431 | 8 | 4.34 | 0.0640 | 49 |
| | female | 5117 | 14395 | 5090 | 107 | 302 | 8 | 5.43 | 0.0703 | 53 |

$AUC_{inf}$: area under the plasma concentration versus time curve from time zero (pre-dose) to infinity; D: dose of midazolam free base; $AUC_{last}$: area under the plasma concentration versus time curve from time zero (pre-dose) to 't' hours (where 't' equals the time point for the last sample on the profile in which the test item was quantified) calculated using the linear/log trapezoidal method; $C_{max}$: maximum plasma concentration of midazolam; $T_{max}$: time point when $C_{max}$ occurred; VzF: Volume of distribution based on the terminal phase. Volume of distribution is determined as $V_z/F = Dose/(\lambda_z * AUC_{(inf)})$; CLF: Clearance: The apparent volume of the central compartment cleared per unit time. Clearance is determined by $CUF = Dose/AUC_{(inf)}$; $T_{1/2}$: terminal plasma half life time.

## Items

The present applicaiton also pertains to the following numbered items.

1. A unit dosage composition in form of an oral thin film comprising midazolam, or a pharmaceutically acceptable salt thereof, a cyclodextrin, and at least one matrix material.

2. The unit dosage composition according to item 1, wherein the amount of midazolam is in the range of from about 0.01 mg to about 50 mg, preferably in the range of from about 0.1 mg to about 20 mg, preferably in the range of from about 0.2 mg to about 15 mg, preferably in the range of from about 0.5 mg to about 10 mg, preferably in the range of from about 1 mg to about 5 mg, more preferably in the range of from about 1.5 mg to about 4.5 mg, yet more preferably in the range of from about 1.8 mg to about 4.3 mg, yet more preferably in the range of from about 2.0 mg to about 4 mg, yet more preferably in the range of from about 2.3 mg to about 3.5 mg, yet more preferably in the range of from about 2.3 mg to about 3.2 mg, yet more preferably in the range of from about 2.4 mg to about 3.1 mg, even more further preferably in the range of from about 2.5 mg to about 3.0 mg, more preferably in the range of from about 2.6 mg to about 2.9 mg wherein the amount of midazolam is determined on the basis of midazolam free base.

3. The unit dosage composition according to item 1 or 2, wherein the pharmaceutically acceptable salt of midazolam is selected from the group consisting of midazolam hydrochloride, and midazolam maleate, preferably the pharmaceutically acceptable salt of midazolam is midazolam hydrochloride.

4. The unit dosage composition according to any one of items 1 to 3, wherein the cyclodextrin is selected from the group consisting of alpha-cyclodextrin, beta-cyclodextrin, gamma-cyclodextrin, and delta-cyclodextrin,

   preferably the cyclodextrin is selected from the group consisting a beta-cyclodextrin, gamma-cyclodextrin, and delta-cyclodextrin,
   more preferably the cyclodextrin is a beta-cyclodextrin,
   even more preferably the cyclodextrin is permethyl-beta-cyclodextrin or hydroxypropyl-beta-cyclodextrin (HpbCD),
   most preferably the cyclodextrin is hydroxypropyl-beta-cyclodextrin.

5. The unit dosage composition according to item 3 or 4, wherein the mass ratio between midazolam free base and hydroxypropyl-beta-cyclodextrin is in the range of from about 1:1 to about 1:10,

   preferably in the range of from about 1:2 to about 1:8,
   preferably in the range of from about 1:3 to about 1:6,
   preferably in the range of from about 1:4 to about 1:5,
   most preferably the mass ratio between midazolam free base and hydroxypropyl-beta-cyclodextrin is about 1:5.2.

6. The unit dosage composition according to item 1 to 5, wherein the at least matrix material is selected from the group consisting of cellulose, cellulose derivatives, acrylic polymer, acrylic co-polymer, and mixtures thereof, preferably the at least matrix material is selected from the group consisting of ethylcellulose, methylcellulose, hydroxypropyl methylcellulose (HPMC), hydroxyethyl cellulose, hydroxypropyl cellulose, carboxymethylcellulose, carboxymethylcellulose sodium salt, microcrystalline cellulose, croscarmellose, cellulose acetate, polyvinylpyrrolidone, polyvinylalcohol (PVA), polyethylene oxide and mixtures thereof, more preferably the at least matrix material is selected from the group consisting of hydroxypropyl methyl cellulose (HPMC), polyvinylalcohol (PVA), and mixtures thereof.

7. The unit dosage composition according to any one of items 1 to 6, wherein the at least one matrix material is a mixture of two matrix materials with different viscosities, preferably wherein the mixture of two matrix materials consists of a first matrix material and a second matrix material, and wherein

> the first matrix material has an about 1.2-fold to about 100-fold higher viscosity than the second matrix material, preferably the first matrix material has an about 2-fold to about 50-fold viscosity than the second matrix material, further preferably the first matrix material has an about 5-fold to about 20-fold viscosity than the second matrix material,
> wherein the viscosity is measured by a viscometer.

8. The unit dosage composition according to any one of items 1 to 7, additionally comprising at least one sweetening agent selected from the group consisting of sucralose and sodium saccharin.

9. The unit dosage composition according to any one of items 1 to 8, additionally comprising glycerol.

10. The unit dosage composition according to any one of items 1 to 9, wherein the unit dosage composition has a pH in the range of from about 2.0 to about 8.0,

> preferably the oral thin film has a pH in the range of from about 2.5 to about 7.0, preferably the oral thin film has a pH in the range of from about 3.0 to about 6.0,
> further preferably the oral thin film has a pH in the range of from about 3.5 to about 5.0.

11. The unit dosage composition according to any one of items 1 to 10 for oral administration, such as buccal administration, or sublingual administration, preferably buccal administration, or sublingual administration.

12. The unit dosage composition according to any one of the previous items for use in the treatment or prevention of febrile seizure, acute seizure, epileptic seizure, such as status epilepticus, fear, such as preoperative anxiolysis to reduce fear, and/or agitation, such as agitation prior to surgery.

13. The unit dosage composition for use according to item 12, wherein the unit dosage composition is administered into the oral cavity of a subject and the therapeutically effective concentration of midazolam in the blood of said subject is reached within less than about 30 minutes,

> preferably is reached within less than about 20 minutes,
> preferably is reached within less than about 15 minutes,
> further preferably the oral thin film is applied in the oral mucosa of a subject and the therapeutically effective concentration of midazolam in the blood of said subject is reached within less than about 10 minutes.

14. The unit dosage composition for use according to item 12 or 13, wherein the unit dosage composition is administered into the oral cavity of a subject and the therapeutically effective concentration of midazolam in the blood of said subject is maintained for more than about 10 minutes and up to about 120 minutes, preferably the therapeutically effective concentration of midazolam in the blood of said subject is maintained for more than about 20 minutes and up to about 90 minutes, further preferably the therapeutically effective concentration of midazolam in the blood of said subject is maintained for more than about 30 minutes and less than about 90 minutes.

15. The unit dosage composition for use according to any one of items 12 to 14, wherein the unit dosage composition is administered into the oral cavity of a subject and the half-life of midazolam in the blood of said subject is in the range of from about 10 minutes to about 240 minutes,

> preferably the half-life of midazolam in the blood of said subject is in the range of from about 20 minutes to about 120 minutes,
> preferably the half-life of midazolam in the blood of said subject is in the range of from about 30 minutes to about 90 minutes,
> further preferably the oral thin film is applied in the oral mucosa of a subject and the half-life of midazolam in the blood of said subject is in the range of from about 40 minutes to about 60 minutes.

16. The unit dosage composition for use according to any one of items 12 to 15, wherein the unit dosage composition is administered into the oral cavity of a subject and the clearance in the blood of said subject is in the range of from about 0.005 L/(min*kg) to about 0.25 L/(min*kg),

preferably is in the range of from about 0.01 L/(min*kg) to about 0.2 L/(min*kg),
further preferably the oral thin film is applied in the oral mucosa of a subject and the clearance in the blood of said subject is in the range of from about 0.01 L/(min*kg) to about 0.15 L/(min*kg).

17. The unit dosage composition for use according to any one of items 12 to 16, wherein the unit dosage composition is administered into the oral cavity of a subject and the distribution volume in the blood of said subject is in the range of from about 0.5 L/kg to about 20 L/kg,

preferably wherein the distribution volume in the blood of said subject is in the range of from about 1 L/kg to about 15 L/kg,
further preferably wherein the distribution volume in the blood of said subject is in the range of from about 2 L/kg to about 10 L/kg.

18. The unit dosage composition for use according to any one of items 12 to 17, wherein the unit dosage composition is administered into the oral cavity of a subject and the profiles of the plasma concentration per dose of said subject are not subjected to a gender effect.

19. A method of treatment or prevention of febrile seizure, acute seizure, epileptic seizure, such as status epilepticus, fear, such as preoperative anxiolysis to reduce fear, and/or agitation, such as agitation prior to surgery, wherein the unit dosage composition according to any one of items 1 to 11 is administered to a subject.

20. Use of the unit dosage composition according to any one of items 1 to 11 for the manufacture of a medicament for the treatment or prevention of febrile seizure, acute seizure, epileptic seizure such as status epilepticus and/or preoperative anxiolysis to reduce fear and agitation prior to surgery.

**Claims**

1. A unit dosage composition in form of an oral thin film comprising midazolam, or a pharmaceutically acceptable salt thereof, a cyclodextrin, and at least one matrix material.

2. The unit dosage composition according to claim 1, wherein the amount of midazolam is in the range of from about 0.01 mg to about 50 mg, preferably in the range of from about 0.1 mg to about 20 mg, preferably in the range of from about 0.2 mg to about 15 mg, preferably in the range of from about 0.5 mg to about 10 mg, preferably in the range of from about 1 mg to about 5 mg, more preferably in the range of from about 1.5 mg to about 4.5 mg, yet more preferably in the range of from about 1.8 mg to about 4.3 mg, yet more preferably in the range of from about 2.0 mg to about 4 mg, yet more preferably in the range of from about 2.3 mg to about 3.5 mg, yet more preferably in the range of from about 2.3 mg to about 3.2 mg, yet more preferably in the range of from about 2.4 mg to about 3.1 mg, even more further preferably in the range of from about 2.5 mg to about 3.0 mg, more preferably in the range of from about 2.6 mg to about 2.9 mg wherein the amount of midazolam is determined on the basis of midazolam free base.

3. The unit dosage composition according to claim 1 or 2, wherein the pharmaceutically acceptable salt of midazolam is selected from the group consisting of midazolam hydrochloride, and midazolam maleate, preferably the pharmaceutically acceptable salt of midazolam is midazolam hydrochloride.

4. The unit dosage composition according to any one of claims 1 to 3, wherein the cyclodextrin is selected from the group consisting of alpha-cyclodextrin, beta-cyclodextrin, gamma-cyclodextrin, and delta-cyclodextrin, preferably the cyclodextrin is selected from the group consisting a beta-cyclodextrin, gamma-cyclodextrin, and delta-cyclodextrin, more preferably the cyclodextrin is a beta-cyclodextrin, even more preferably the cyclodextrin is permethyl-beta-cyclodextrin or hydroxypropyl-beta-cyclodextrin (HpbCD), most preferably the cyclodextrin is hydroxypropyl-beta-cyclodextrin.

5. The unit dosage composition according to claim 3 or 4, wherein the mass ratio between midazolam free base and hydroxypropyl-beta-cyclodextrin is in the range of from about 1:1 to about 1:10, preferably in the range of from about 1:2 to about 1:8, preferably in the range of from about 1:3 to about 1:6,

preferably in the range of from about 1:4 to about 1:5,
most preferably the mass ratio between midazolam free base and hydroxypropyl-beta-cyclodextrin is about 1:5.2.

6. The unit dosage composition according to claim 1 to 5, wherein the at least matrix material is selected from the group consisting of cellulose, cellulose derivatives, acrylic polymer, acrylic co-polymer, and mixtures thereof, preferably the at least matrix material is selected from the group consisting of ethylcellulose, methylcellulose, hydroxypropyl methylcellulose (HPMC), hydroxyethyl cellulose, hydroxypropyl cellulose, carboxymethylcellulose, carboxymethylcellulose sodium salt, microcrystalline cellulose, croscarmellose, cellulose acetate, polyvinylpyrrolidone, polyvinylalcohol (PVA), polyethylene oxide and mixtures thereof, more preferably the at least matrix material is selected from the group consisting of hydroxypropyl methyl cellulose (HPMC), polyvinylalcohol (PVA), and mixtures thereof, and/or
wherein the at least one matrix material is a mixture of two matrix materials with different viscosities, preferably wherein the mixture of two matrix materials consists of a first matrix material and a second matrix material, and wherein
the first matrix material has an about 1.2-fold to about 100-fold higher viscosity than the second matrix material, preferably the first matrix material has an about 2-fold to about 50-fold viscosity than the second matrix material, further preferably the first matrix material has an about 5-fold to about 20-fold viscosity than the second matrix material, wherein the viscosity is measured by a viscometer.

7. The unit dosage composition according to any one of claims 1 to 6, additionally comprising at least one sweetening agent selected from the group consisting of sucralose and sodium saccharin; and/or
additionally comprising glycerol.

8. The unit dosage composition according to any one of claims 1 to 7, wherein the unit dosage composition has a pH in the range of from about 2.0 to about 8.0,
preferably the oral thin film has a pH in the range of from about 2.5 to about 7.0, preferably the oral thin film has a pH in the range of from about 3.0 to about 6.0, further preferably the oral thin film has a pH in the range of from about 3.5 to about 5.0.

9. The unit dosage composition according to any one of claims 1 to 8 for oral administration, such as buccal administration, or sublingual administration, preferably buccal administration, or sublingual administration.

10. The unit dosage composition according to any one of the previous claims for use in the treatment or prevention of febrile seizure, acute seizure, epileptic seizure, such as status epilepticus, fear, such as preoperative anxiolysis to reduce fear, and/or agitation, such as agitation prior to surgery.

11. The unit dosage composition for use according to claim 10, wherein the unit dosage composition is administered into the oral cavity of a subject and the therapeutically effective concentration of midazolam in the blood of said subject is reached within less than about 30 minutes,
preferably is reached within less than about 20 minutes,
preferably is reached within less than about 15 minutes,
further preferably the oral thin film is applied in the oral mucosa of a subject and the therapeutically effective concentration of midazolam in the blood of said subject is reached within less than about 10 minutes; and/or
wherein the unit dosage composition is administered into the oral cavity of a subject and the therapeutically effective concentration of midazolam in the blood of said subject is maintained for more than about 10 minutes and up to about 120 minutes,
preferably the therapeutically effective concentration of midazolam in the blood of said subject is maintained for more than about 20 minutes and up to about 90 minutes, further preferably the therapeutically effective concentration of midazolam in the blood of said subject is maintained for more than about 30 minutes and less than about 90 minutes.

12. The unit dosage composition for use according to any one of claims 10 or 11, wherein the unit dosage composition is administered into the oral cavity of a subject and the half-life of midazolam in the blood of said subject is in the range of from about 10 minutes to about 240 minutes,
preferably the half-life of midazolam in the blood of said subject is in the range of from about 20 minutes to about 120 minutes,
preferably the half-life of midazolam in the blood of said subject is in the range of from about 30 minutes to about 90 minutes,
further preferably the oral thin film is applied in the oral mucosa of a subject and the half-life of midazolam in the blood of said subject is in the range of from about 40 minutes to about 60 minutes.

13. The unit dosage composition for use according to any one of claims 10 to 12, wherein the unit dosage composition is administered into the oral cavity of a subject and the clearance in the blood of said subject is in the range of from about 0.005 L/(min*kg) to about 0.25 L/(min*kg),
preferably is in the range of from about 0.01 L/(min*kg) to about 0.2 L/(min*kg), further preferably the oral thin film is applied in the oral mucosa of a subject and the clearance in the blood of said subject is in the range of from about 0.01 L/(min*kg) to about 0.15 L/(min*kg).

14. The unit dosage composition for use according to any one of claims 10 to 13, wherein the unit dosage composition is administered into the oral cavity of a subject and the distribution volume in the blood of said subject is in the range of from about 0.5 L/kg to about 20 L/kg,
preferably wherein the distribution volume in the blood of said subject is in the range of from about 1 L/kg to about 15 L/kg,
further preferably wherein the distribution volume in the blood of said subject is in the range of from about 2 L/kg to about 10 Ukg.

15. The unit dosage composition for use according to any one of claims 10 to 14, wherein the unit dosage composition is administered into the oral cavity of a subject and the profiles of the plasma concentration per dose of said subject are not subjected to a gender effect.

Fig. 1

**Fig. 2**

**Fig. 3**

Fig. 4

Fig. 5

**Fig. 6**

Europäisches
Patentamt

European
Patent Office

Office européen
des brevets

**EUROPEAN SEARCH REPORT**

Application Number

EP 23 16 7083

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/025890 A1 (SWIPP AB [SE]) 2 March 2023 (2023-03-02) | 2,3,6, 8-15 | INV. A61K9/00 |
| Y | * abstract * * claim 1 * * composition A12; example 10 * * page 6, line 10 – line 11 * * page 9, line 31 – line 34 * * page 13, line 19 – line 20 * * figure 2 * | 1,4 | A61K31/5517 A61K47/10 A61K47/32 A61K47/38 A61K47/40 A61P25/08 A61P25/20 A61P25/22 |
| Y | RUUT KAARTAMA ET AL: "The effect of hydroxypropyl-beta-cyclodextrin and sucrose on the sublingual absorption of midazolam in rabbits", EUROPEAN JOURNAL OF PHARMACEUTICS AND BIOPHARMACEUTICS, ELSEVIER SCIENCE PUBLISHERS B.V., AMSTERDAM, NL, vol. 81, no. 1, 23 January 2012 (2012-01-23), pages 178-183, XP028484406, ISSN: 0939-6411, DOI: 10.1016/J.EJPB.2012.01.014 [retrieved on 2012-01-31] * abstract * * page 180, right-hand column * * page 182 – paragraph 3.3 * * table 1 * | 1,4 | |
| X | WO 01/30391 A2 (FARMARC NEDERLAND BV [NL]; PENKLER LAWRENCE JOHN [ZA]) 3 May 2001 (2001-05-03) * figure 1 * | 5 | TECHNICAL FIELDS SEARCHED (IPC) A61K |

−/−−

The present search report has been drawn up for all claims

| Place of search | Date of completion of the search | Examiner |
|---|---|---|
| Munich | 16 August 2023 | Hidaoui, Nadia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 23 16 7083**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LOFTSSON ET AL: "Cyclodextrins and their pharmaceutical applications", INTERNATIONAL JOURNAL OF PHARMACEUTICS, ELSEVIER, NL, vol. 329, no. 1-2, 20 December 2006 (2006-12-20), pages 1-11, XP005809167, ISSN: 0378-5173, DOI: 10.1016/J.IJPHARM.2006.10.044 * page 6 – paragraph 2.3.4 * | 1 | |
| X | MONTERO-PADILLA SOLEDAD ET AL: "Buccal Dosage Forms: General Considerations for Pediatric Patients", AAPS PHARMSCITECH, SPRINGER US, NEW YORK, vol. 18, no. 2, 14 June 2016 (2016-06-14), pages 273-282, XP036139459, DOI: 10.1208/S12249-016-0567-2 [retrieved on 2016-06-14] * page 179, right-hand column * | 7 | |

TECHNICAL FIELDS
SEARCHED (IPC)

The present search report has been drawn up for all claims

| Place of search | Date of completion of the search | Examiner |
|---|---|---|
| Munich | 16 August 2023 | Hidaoui, Nadia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 16 7083**

This annex lists the patent family members relating to the patent documents cited in the above-mentioned European search report.
The members are as contained in the European Patent Office EDP file on
The European Patent Office is in no way liable for these particulars which are merely given for the purpose of information.

**16-08-2023**

| Patent document cited in search report | | Publication date | Patent family member(s) | | Publication date |
|---|---|---|---|---|---|
| WO 2023025890 | A1 | 02-03-2023 | NONE | | |
| WO 0130391 | A2 | 03-05-2001 | AU | 7937500 A | 08-05-2001 |
| | | | WO | 0130391 A2 | 03-05-2001 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 59467-70-8
- *CHEMICAL ABSTRACTS,* 59467-96-8
- *CHEMICAL ABSTRACTS,* 59467-94-6
- *CHEMICAL ABSTRACTS,* 10016-20-3
- *CHEMICAL ABSTRACTS,* 7585-39-9
- *CHEMICAL ABSTRACTS,* 17465-86-0
- *CHEMICAL ABSTRACTS,* 55216-11-0
- *CHEMICAL ABSTRACTS,* 128446-35-5
- *CHEMICAL ABSTRACTS,* 9004-65-3
- *CHEMICAL ABSTRACTS,* 9002-89-5